(19) Europäisches Patentamt
European Patent Office
Office européen des brevets

(11) EP 3 384 079 B1

(12) **EUROPEAN PATENT SPECIFICATION**

(45) Date of publication and mention of the grant of the patent:
**15.04.2020 Bulletin 2020/16**

(21) Application number: **16826786.2**

(22) Date of filing: **05.12.2016**

(51) Int Cl.:
***D04H 1/728*** *(2012.01)* ***A61L 2/18*** *(2006.01)*
***C11D 3/386*** *(2006.01)* ***C11D 3/39*** *(2006.01)*
***C12P 7/40*** *(2006.01)* ***C11D 17/04*** *(2006.01)*
***D01D 5/00*** *(2006.01)*

(86) International application number:
**PCT/US2016/064927**

(87) International publication number:
**WO 2017/096354 (08.06.2017 Gazette 2017/23)**

(54) **A FIBROUS CONSTRUCT AND METHODS RELATING THERETO**

FASERKONSTRUKT UND DAMIT ZUSAMMENHÄNGENDES VERFAHREN

CONSTRUCTION FIBREUSE ET PROCÉDÉS ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.12.2015 US 201562262625 P**
**03.12.2015 US 201562262631 P**

(43) Date of publication of application:
**10.10.2018 Bulletin 2018/41**

(73) Proprietor: **DuPont US Holding, LLC**
**Wilmington, DE 19805 (US)**

(72) Inventor: **SAQUING, Carl**
**Newark, Delaware 19713 (US)**

(74) Representative: **Dehns**
**St. Bride's House**
**10 Salisbury Square**
**London EC4Y 8JD (GB)**

(56) References cited:
**WO-A1-2009/067279** **WO-A1-2010/062745**
**US-B2- 7 582 247**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF DISCLOSURE

This disclosure relates generally to a fibrous construct and more specifically to perhydrolase encapsulated in fibers.

### BACKGROUND OF THE DISCLOSURE

Enzymes are commonly required as catalysts in various industries. However, enzymes have limited application and shelf life due to their instability. Enzyme activity generally decreases during storage or processing making their use in many processes difficult.

Enzymes are often supplied in liquid formulations. Liquid formulations are preferred in many cases for several reasons, including solubility, convenience in handling (e.g., dispensing, pouring, pumping or mixing), and compatibility with existing manufacturing processes, which are typically aqueous processes. Liquid formulations for the co-delivery of enzymes and substrates are disclosed in WO 2010/062745.

Specific perhydrolase enzymes capable of producing peracids, and suitable formulations of such enzymes, are disclosed in WO 2009/067279.

Enzymes are often biochemically less stable in aqueous liquids. When an enzyme is added to an aqueous medium without steps taken to stabilize the enzyme, the enzyme typically is rapidly denatured in the water. Enzymes may hydrolyze in water and often will degrade itself or other enzymes that may be present. In the aqueous state, undesirable reactions (e.g., proteolysis, premature catalytic conversion of substrates, loss of cofactors, oxidation) often occur at unacceptable rates. Aqueous enzyme formulations can also exhibit signs of physical instability, including the formation of precipitates, crystals, gels, or turbidity, during extended storage. Consequently, a loss of enzyme activity is observed over time.

Thus there is a need for enzymes to retain their activity for long periods of time (shelf-storage) and particularly for aqueous compositions. It is desirable that the enzyme be physically isolated from its substrate until the reaction is desired.

### SUMMARY

The present disclosure is directed to a fibrous construct comprising:

a) an ester substrate;
b) a first web comprising a plurality of first water soluble fibers and a perhydrolase, where the perhydrolase is encapsulated in the first water soluble fibers and is present in an amount from 0.1 to 40 wt% based on the total weight of the first web;
c) a second web comprising a plurality of second water soluble fibers and an oxidizing agent, where the oxidizing agent is encapsulated in the second water soluble fibers; and

where the first water soluble fibers and the second water soluble fibers are solution spun water soluble fibers.

The present disclosure is also directed to a fibrous construct comprising:

a) an ester substrate;
b) a first web comprising a plurality of first water soluble fibers and a perhydrolase, where the perhydrolase is encapsulated in the first water soluble fibers and is present in an amount from 0.1 to 40 wt% based on the total weight of the first web;
c) a second web comprising a plurality of second water soluble fibers and hydrogen peroxide, where the hydrogen peroxide is complexed on to at least a portion of the second web;

where the second water soluble fibers are polyvinyl pyrrolidone or copolymers thereof; and
wherein the first water soluble fibers and the second water soluble fibers are solution spun water soluble fibers.

### DETAILED DESCRIPTION

#### Definitions

As used herein, the terms "comprises," "comprising," "includes," "including," "has," "having" or any other variation

thereof, are intended to cover a non-exclusive inclusion. For example, a method, process, article, or apparatus that comprises a list of elements is not necessarily limited only to those elements but may include other elements not expressly listed or inherent to such method, process, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, use of "a" or "an" are employed to describe elements and components of the invention. This is done merely for convenience and to give a general sense of the invention. This description should be read to include one or at least one and the singular also includes the plural unless it is obvious that it is meant otherwise.

The term "substrate" as used herein is intended to mean a substance (e.g., a chemical compound) on which an enzyme performs its catalytic activity to generate a product.

The term "fiber" or "fibers" as used herein is intended to mean any structure (e.g. a matrix or coated structure) that has an aspect ratio (L/D) of at least 5.

The term "web", "fiber mat", "fiber matrices" as used herein is intended to mean a structure comprising more than one fiber, where the fibers are in proximity or contact with one another at one or more points.

The term "fragmented web" or "fragmented non-woven web" or "fragmented woven web" as used herein is intended to mean a web of fibers broken apart from a larger web either mechanically (for example by milling) or chemically where the fragmentation is effected through a chemical agent (example by partial dissolution) or a combination of mechanical and chemical means. The fragmented web may be in the form of powders or particles or powdery particulates to the naked eye, but when seen using a high resolution microscope (for example scanning electron microscope), the fragmented web appears fibrous in morphology.

The term "solution spun" or "solution spinning" as used herein is intended to mean the formation of fibers by extruding a solution of a polymer composition from a spinneret or spin pack to form fine streams of fluid and includes both dry spinning and wet spinning. With dry spinning, the polymer solution jet or jets come across a stream of inert gas typically air and evaporates the solvent. Wet spinning is similar to dry spinning, except that the polymer solution jet or jets come across a stream of liquid solvent or solution that is miscible with the polymer solvent but does not dissolve the polymer. The term "solution spun" or "solution spinning" is intended to also include electrospinning from polymer solution or electroblowing from polymer solution or centrifugal spinning from polymer solution.

The term "encapsulated", "encapsulate," "encapsulates," "encapsulation" or other similar terminology as used herein refers to at least partially or completely surrounding or associating an active substance (e.g. an enzyme or oxidizing agent) with another material (e.g., polymeric matrix) to prevent or control the release of active, for example, within an aqueous composition.

The term "encapsulation efficiency" as used herein means the percent of enzyme solids (active and inactive) by mass that gets incorporated in the delivery system relative to the total mass of enzyme solids contained in the starting spinning solution.

The term "enzyme payload" or "enzyme activity" as used herein means the concentration in mass of active enzyme that is encapsulated in the delivery system. It can be expressed in activity units, but preferably is expressed gravimetrically in units of mg/g (mg active enzyme per gram of delivery system).

The term "encapsulation yield" is the mass percentage of active enzyme that is recovered from the delivery system after encapsulation. An encapsulation yield of 100% is known as the "theoretical payload" and implies no inactivation of the enzyme that is encapsulated.

The term "absorbed" for the purpose of the present disclosure is intended to mean taken up into fibers, occupying or filling spaces between fibers of a web or on the surface of the fibers and can be used interchangeably with "immobilized" in the narrower sense of physical immobilization as opposed to chemical or covalent immobilization.

The term "suspension" or "suspended" as used herein refers to a two phase system where a discontinuous solid phase (e.g., fibers) is dispersed within a continuous liquid phase.

The term "soluble" or "solubility" for the purpose of the present disclosure is intended to mean completely in solution at the molecular level or partially in solution. "Partially in solution" means the amount or fraction of the material (e.g., polymer) present in a supernatant resulting from centrifugation. Solubility can be measured, for example, by measuring the material (e.g., polymer) that remains in the supernatant after centrifuging an aqueous suspension containing the material (for example, the material can be a plurality of solution spun fibers, such as a fiber mat with or without enzyme).

The term "dissolution" or "dissolve" or similar terminology used herein refers to a process where solution spun fibers or fiber delivery system becomes soluble.

The term "complexed" as used herein is intended to mean the formation of hydrogen bonds between two or more molecules. For example, hydrogen bonds can form between the vinyl pyrrolidone (VP) side group of PVP and hydrogen peroxide. The vinyl pyrrolidone side group is a five member lactam ring with an amide carbonyl that is a strong hydrogen acceptor. Since hydrogen peroxide is a strong hydrogen donor, it will form a stable complex with vinyl pyrrolidone. Molecularly, complexes of hydrogen peroxide and VP can form for example with one molecule hydrogen peroxide to one molecule VP (1:1), and one molecule of hydrogen peroxide to two molecules of VP (1:2) as seen in the complex structures below. This complexation with hydrogen peroxide then can be formed in copolymers of PVP where the vinyl pyrrolidone as a side group is present.

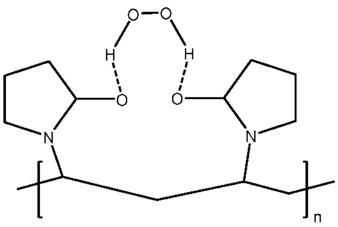

,

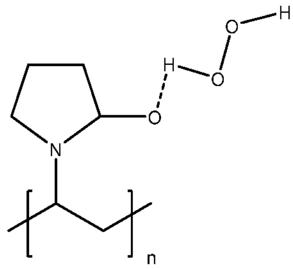

When an amount, concentration, or other value or parameter is given as either a range, preferred range or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. Numerical values are to be understood to have the precision of the number of significant figures provided. For example, the number 1 shall be understood to encompass a range from 0.5 to 1.4, whereas the number 1.0 shall be understood to encompass a range from 0.95 to 1.04, including the end points of the stated ranges. It is not intended that the scope of the invention be limited to the specific values recited when defining a range.

In describing certain polymers, it should be understood that sometimes applicants are referring to the polymers by the monomers used to make them or the amounts of the monomers used to make them. While such a description may not include the specific nomenclature used to describe the final polymer or may not contain product-by-process terminology, any such reference to monomers and amounts should be interpreted to mean that the polymer is made from those monomers, unless the context indicates or implies otherwise.

The materials, methods, and examples herein are illustrative only and, except as specifically stated, are not intended to be limiting. Although methods and materials similar or equivalent to those described herein can be used, suitable methods and materials are described herein.

The present disclosure is directed to a fibrous construct comprising:

a) an ester substrate;

b) a first web comprising a plurality of first water soluble fibers and a perhydrolase, where the perhydrolase is encapsulated in the first water soluble fibers and is present in an amount from 0.1 to 40 wt% based on the total weight of the first web;

c) a second web comprising a plurality of second water soluble fibers and an oxidizing agent, where the oxidizing agent is encapsulated in the second water soluble fibers; and

wherein the first water soluble fibers and the second water soluble fibers are solution spun water soluble fibers.

In some embodiments, the oxidizing agent is hydrogen peroxide and the hydrogen peroxide is complexed onto at least a portion of the second web comprising a plurality of second water soluble fibers where the second water soluble fibers are polyvinyl pyrrolidone or copolymers thereof and where the first and second water soluble fibers are solution spun water soluble fibers.

In some embodiments, the fibrous construct comprises a third web. The third web comprises a plurality of third water soluble fibers.

<u>Fibers</u>

The fibers of the present disclosure are produced using a water soluble resin or mixtures of water soluble resins. The fibers can be produced using a mixture of at least one water soluble resin and a non-water soluble resin such that amounts of each are tailored to achieve the desired solubility (quick release or controlled release).

In some embodiments, the first water soluble fibers and the second water soluble fibers are independently selected from methylcellulose, hydroxypropyl methylcellulose, guar gum, alginate, polyvinyl pyrrolidone (PVP), polyethylene oxide, polyvinyl alcohol (PVA), pullulan, polyaspartic acid, polyacrylic acid, polylactic acid, copolymers thereof or mixtures thereof.

In some embodiments, the first water soluble fibers, the second water soluble fibers and the third water soluble fibers are independently selected from methylcellulose, hydroxypropyl methylcellulose, guar gum, alginate, polyvinyl pyrrolidone, polyethylene oxide, polyvinyl alcohol, pullulan, polyaspartic acid, polyacrylic acid, copolymers thereof or mixtures thereof.

In some embodiments, the first water soluble fibers and the second water soluble fibers are independently selected from polyvinyl pyrrolidone, polyvinyl alcohol, pullulan or mixtures thereof.

In some embodiments, the first water soluble fibers, the second water soluble fibers and the third water soluble fibers are independently selected from polyvinyl pyrrolidone, polyvinyl alcohol, pullulan or mixtures thereof. In some embodiments the second water soluble fibers are formed from polyvinyl pyrrolidone or copolymers thereof.

In one embodiment, the first water soluble fibers are fully hydrolyzed polyvinyl alcohol. Fully hydrolyzed is intended to mean 98% hydrolyzed or greater. In one embodiment, the first water soluble fibers have a crystallinity of from 20 to 54%. In one embodiment, the first water soluble fibers are fully hydrolyzed polyvinyl alcohol having a crystallinity of from 20 to 54%. In another embodiment, the first water soluble fibers have a crystallinity less than 35%. In another embodiment, the first water soluble fibers are fully hydrolyzed polyvinyl alcohol having a crystallinity less than 35%.

In yet another embodiment, the first water soluble fibers and the second water soluble fibers are fully hydrolyzed polyvinyl alcohol. In yet another embodiment, the first water soluble fibers and the second water soluble fibers have a crystallinity of 20 to 54%. In yet another embodiment, the first water soluble fibers and the second water soluble fibers are fully hydrolyzed polyvinyl alcohol having a crystallinity of 20 to 54%. In another embodiment, the first water soluble fibers and the second water soluble fibers are fully hydrolyzed polyvinyl alcohol having a crystallinity between and optionally including any two of the following: 20, 25, 30, 35, 40, 45, 50 and 54%. In yet another embodiment, the first water soluble fibers and the second water soluble fibers have a crystallinity less than 35%. In yet another embodiment, the first water soluble fibers and the second water soluble fibers are fully hydrolyzed polyvinyl alcohol having a crystallinity less than 35%. In yet another embodiment, the first water soluble fibers and the second water soluble fibers are fully hydrolyzed polyvinyl alcohol having a crystallinity less than 30%.

In yet another embodiment, the first water soluble fibers, the second water soluble fibers and the third water soluble fibers are fully hydrolyzed polyvinyl alcohol.

In yet another embodiment, the first water soluble fibers, the second water soluble fibers and the third water soluble fibers have a crystallinity from 20 to 54%. In yet another embodiment, the first water soluble fibers, the second water soluble fibers and the third water soluble fibers are fully hydrolyzed polyvinyl alcohol having a crystallinity from 20 to 54%. In another embodiment, the first water soluble fibers, the second water soluble fibers and the third water soluble fibers are fully hydrolyzed polyvinyl alcohol having a crystallinity between and optionally including any two of the following: 20, 25, 30, 35, 40, 45, 50 and 54%. In yet another embodiment, the first water soluble fibers, the second water soluble fibers and the third water soluble fibers have a crystallinity less than 35%. In yet another embodiment, the first water soluble fibers, the second water soluble fibers and the third water soluble fibers are fully hydrolyzed polyvinyl alcohol having a crystallinity less than 35%. In yet another embodiment, the first water soluble fibers, the second water soluble fibers and the third water soluble fibers are fully hydrolyzed polyvinyl alcohol having a crystallinity less than 30%.

In some embodiments where the second water soluble fibers are polyvinyl pyrrolidone or a copolymer thereof, the first water soluble fibers and third water soluble fibers may be polyvinyl alcohol such as fully hydrolyzed polyvinyl alcohol. In such embodiments the polyvinyl alcohol may in some cases have a crystallinity of less than 30% or between and optionally including any two of the following: 20, 25, 30, 35, 40, 45, 50 and 54%.

In some embodiments, the first water soluble fibers are a polysaccharide or any other naturally occurring resin that is water soluble.

Some enzymes, as well as other active agents, cannot withstand the high temperatures necessary for melt spinning fibers. Thus, at least the first water soluble fibers of the present disclosure are solution spun fibers. In some embodiments, the first and second water soluble fibers are solution spun and in other embodiments the first, second and third water soluble fibers are solution spun.

In some embodiments, the solution spun fibers are dry spun. In some embodiments, the solution spun fibers are wet spun. In some embodiments, the solution spun fibers are electrospun. In some embodiments, the solution spun fibers are centrifugally spun. In yet another embodiment, the solution spun fibers are electroblown. The terms "electroblown" or "electroblowing" or "electro-blown spinning" may be used interchangeably and is intended to mean where a polymer-enzyme solution is fed towards a spinning nozzle, discharging the polymer solution via the spinning nozzle or spinneret, which is charged with a high voltage, while injecting compressed air via the lower end of the spinning nozzle, and collecting fiber spun, typically in the form of a web. Examples of techniques for electroblowing are disclosed in for example US Patent Nos. 7,618,579 and 7,582,247.

In some embodiments, the first water soluble fibers and the second water soluble fibers are electroblown water soluble fibers. In some embodiments, the first water soluble fibers, the second water soluble fibers and the third water soluble fibers are electroblown water soluble fibers.

In some embodiments, at least a portion of the first water soluble fibers or at least a portion of the second water

soluble fibers or at least a portion of the third water soluble fibers have an average diameter of 25 microns or less. In another embodiment, each have an average diameter of 25 microns or less.

In some embodiments, at least a portion of the first water soluble fibers or at least a portion of the second water soluble fibers or at least a portion of the third water soluble fibers have an average diameter of 20 microns or less. In another embodiment, each have an average diameter of 20 microns or less.

In some embodiments, at least a portion of the first water soluble fibers or at least a portion of the second water soluble fibers or at least a portion of the third water soluble fibers have an average diameter of 5 microns or less. In another embodiment, each have an average diameter of 5 microns or less.

In another embodiment, at least a portion of the first water soluble fibers or at least a portion of the second water soluble fibers or at least a portion of the third water soluble fibers have an average diameter of 1 microns or less. In another embodiment, each have an average diameter of 1 microns or less.

In another embodiment, at least a portion of the first water soluble fibers or at least a portion of the second water soluble fibers or at least a portion of the third water soluble fibers have an average diameter of 0.9 microns or less. In another embodiment, each has an average diameter of 0.9 microns or less.

In another embodiment, at least a portion of the first water soluble fibers or at least a portion of the second water soluble fibers or at least a portion of the third water soluble fibers have an average diameter of 0.5 microns or less. In another embodiment, each have an average diameter of 0.5 microns or less.

In another embodiment, at least a portion of the first water soluble fibers or at least a portion of the second water soluble fibers or at least a portion of the third water soluble fibers have an average diameter of 0.4 microns or less. In another embodiment, each have an average diameter of 0.4 microns or less.

In another embodiment, at least a portion of the first water soluble fibers or at least a portion of the second water soluble fibers or at least a portion of the third water soluble fibers have an average diameter of 0.3 microns or less. In another embodiment, each have an average diameter of 0.3 microns or less.

In some embodiments, the first water soluble fibers or the second water soluble fibers or the third water soluble fibers have an average fiber diameter between and optionally including any two of the following: 0.2, 0.3, 0.4, 0.5, 0.6, 0.7, 0.8, 0.9 and 1.0 microns. In some embodiments, the first water soluble fibers or the second water soluble fibers or the third water soluble fibers have an average fiber diameter from 0.2 to 1.0 microns. In some embodiments, each have an average fiber diameter between and optionally including any two of the following: 0.2, 0.3, 0.4, 0.5, 0.6, 0.7, 0.8, 0.9 and 1.0 microns.

For the aforesaid fiber diameters, the process for making the water soluble fibers is preferably by a solution spinning process as previously described. One skilled in the art will recognize however that depending on the stability of the active to be encapsulated and the water soluble polymer resin, it may be possible to form fibers using other processes such as by a centrifugal melt spinning process.

## Fiber solubility

It was discovered that polyvinyl alcohol (PVA) fiber matrices (also referred to herein as a "web" or "fiber mat") with and without enzyme can have different solubility and fragmentation behavior in water and water-organic solvent mixtures including water-propylene glycol and water-ethanol mixtures at different water content. These behaviors are related to fiber size and fiber crystallinity and have an impact on enzyme leakage.

PVA solubility in water has been known to be affected by degree of hydrolysis of PVA (Briscoe B, Luckham P, Zhu S. The effects of hydrogen bonding upon the viscosity of aqueous poly(vinyl alcohol) solutions. Polymer. 2000;41:3851-3860). As the degree of hydrolysis is increased, the amount of the hydrophobic acetate groups are decreased, hence the PVA solubility is increased. In general, PVA with a degree of hydrolysis below 70 %, becomes insoluble. As you increase above 70 % degree of hydrolysis, PVA solubility increases up to a maximum (ca. 90 %) at which the PVA solubility starts to decrease due to overpowering effect of strong inter and intra chain hydrogen bonding making the polymer highly crystalline. It has been found that for enzymes encapsulated in a PVA fiber mat, release of the encapsulated enzyme can be controlled by PVA polymer crystallinity and PVA fiber size.

The % crystallinity of the fiber can be determined using dynamic scanning calorimetry (DSC) according to techniques known to those skilled in the art. The crystallinity of PVA fiber and powder was determined according to the procedure described in Example 7 using a Q1000 Modulated DSC from TA Instruments.

In some embodiments, the solution spun fibers have a crystallinity from 20% to 54%. In some embodiments, the solution spun fibers have a crystallinity from 20% to 54% and the water soluble polymeric resin is a fully hydrolyzed polyvinyl alcohol.

In some embodiments, the solution spun fibers have a crystallinity less than 35%. In some embodiments, the solution spun fibers have a crystallinity of less than 35% and the water soluble polymeric resin is a fully hydrolyzed polyvinyl alcohol.

Examples of suitable PVA that may be used for solution spun fibers useful in the present application include

ELVANOL 80-18 and ELVANOL 70-30. ELVANOL 80-18 is a 98 to 98.8 % hydrolysed copolymer of PVA (polyvinyl alcohol) and another monomer and available from Kuraray Co., Ltd. ELVANOL 70-03 is a 98 to 98.8 % hydrolysed PVA (polyvinyl alcohol) and also available from Kuraray Co., Ltd.

As further described in Example 7, the solubility behavior in water of the PVA fiber mats in comparison to PVA powders as obtained from the manufacturer was determined by visual inspection and quantitatively. Both ELVANOL 70-03 and ELVANOL 80-18 as obtained from the manufacturer do not dissolve or disintegrate in water at room and cold temperature.

When transformed into fibers, it was surprisingly discovered that there was rapid fragmentation and eventual visual disappearance of the polyvinyl alcohol fibers in water.

The solubility behavior of the fibers was determined quantitatively according the procedure described in Example 7 where a known amount of fiber was placed in a solution consisting of water and propylene glycol ranging from: 0 wt% water/100 wt% propylene glycol to 100 wt% water/0 wt% propylene glycol at a controlled temperature and fiber concentration. The solution was held for a desired time at a desired temperature and was then centrifuged and the supernatant was analyzed for PVA content using the spectrophotometric method described in the published journal article "Simple spectrophotometric method for determination of polyvinyl alcohol in different types of wastewater, L. Procházková, Y. Rodríguez-Muñoz, J. Procházka, J. Wannera, 2014, Intern. J. Environ. Anal. Chem., 94, 399-410". This method is based on complexation with iodine according to the Pritchard method that has been previously described in earlier publications including the following: I.F. Aleksandrovich and L.N. Lyubimova, Fibre Chem. 24, 156 (1993); D.P. Joshi, Y.L. Lan-Chun-Fung and J.G. Pritchard, Anal. Chim. Acta. 104, 153 (1979); Y. Morishima, K. Fujisawa and S. Nozakura, Polym. J. 10, 281 (1978); J.G. Pritchard and D.A. Akintola, Talanta. 19, 877 (1972).

As described in Example 7, PVA fiber mats placed in water were no longer visible with the naked eye within 2 minutes at room temperature (~25°C), between 2.5 to 5 min at 15°C, between 3 to 7 min at 10°C, between 5 to 10 min at 5°C and between 10 to 20 min at close to 0°C. Not being bound to any particular theory, two possible factors may have contributed to this: the high surface area of the PVA fibers compared to the PVA powders and the decreased crystallinity of the PVA chains in the fibers. The fiber mat solubility properties can advantageously be used in cold water cleaning technology including cold water laundry detergent applications.

In some embodiments, the solution spun fibers have a solubility of 7.7 mg/mL or less in water at a temperature from 30 to 0 degrees C. In some embodiments, solution spun fibers of fully hydrolyzed polyvinyl alcohol have a solubility of 7.7 mg/mL or less in water at a temperature from 30 to 0 degrees C.

In some embodiments, the first water soluble fibers and the second water soluble fibers are solution spun water soluble fibers having a solubility of 7.7 mg/mL or less in water at temperatures of 30 to 0 degrees C.

In some embodiments, the first water soluble fibers, the second water soluble fibers and the third water soluble fibers are solution spun water soluble fibers having a solubility of 7.7 mg/mL or less in water at temperatures of 30 to 0 degrees C.

## Enzyme

The fibrous construct of the present disclosure contains a perhydrolase which is encapsulated in a web comprising a plurality of water soluble fibers. In some embodiments, the perhydrolase is a perhydrolase variant. Variant proteins differ from a parent protein and/or from one another by a small number of amino acid residues. In some embodiments, the number of different amino acid residues is any of about 1, 2, 3, 4, 5, 10, 20, 25, 30, 35, 40, 45, or 50. In some embodiments, variants differ by about 1 to about 10 amino acids. In some embodiments, related proteins, such as variant proteins, comprise any of at least about 35%, 40%, 45%, 50%, 55%,60%, 65%, 70%, 75%, 80%, 85%, 90%, 95%, 97%, 98%, 99%, or 99.5% amino acid sequence identity.

The perhydrolase is encapsulated in the first water soluble fibers. The perhydrolase is present in an amount from 0.1 to 40 wt% based on the total weight of the first web. In some embodiments, the perhydrolase is present in an amount between and including any two of the following: 0.1, 0.5, 1, 2, 3, 4, 5, 10, 15, 20, 25, 30, 35 and 40 wt% based on the total weight of the first web. In some embodiments, the perhydrolase is present in an amount from 0.1 to 30 wt% based on the total weight of the first web. In some embodiments, the perhydrolase is present in an amount from 0.1 to 15 wt% based on the total weight of the first web. In some embodiments, the perhydrolase is present in an amount between and including any two of the following: 0.1, 0.17, 0.5, 1.0, 1.7, 2.0, 2.2, 2.5 and 3.5 wt% based on the total weight of the first web.

Encapsulation efficiency is the percent of mass of enzyme solid (active and inactive) that is encapsulated in the enzyme delivery system based on the total mass of enzyme solid (active and inactive) contained in the starting solution for spinning. Encapsulation efficiency may be calculated as:

$$\text{Encapsulation Efficiency (\%)} = 100 \times \frac{\text{(Total amount of enzyme in fiber mat)}}{\text{(Total amount of enzyme in spin solution)}}$$

A higher encapsulation efficiency percentage implies a greater amount of the starting enzyme was encapsulated. For example, 100% efficiency means that all the enzyme in the starting solution was encapsulated in the fibers. As is further described in Example 1 herein, it was found that the encapsulation efficiency may be 95% ± 10%. It is believed that the difference between 100% efficiency versus what was observed could be within statistical or random error. Thus, the method of enzyme encapsulation described herein may be more efficient than conventional encapsulation methods for enzymes like spray drying and fluidized bed processes. In some embodiments, the encapsulation efficiency of the perhydrolase is from 80 to 100%.

The distribution of the perhydrolase in the fiber mat was determined by directly measuring the protein content using the sodium dodecyl sulfate polyacrylamide gel electrophoresis (SDS-PAGE) protocol or the Laemmli method (Laemmli, U. K. (1970)) according to the procedure described in Example 1. The analysis in Example 1 showed that the enzyme distribution of perhydrolase in the fiber mat was highly uniform.

Enzyme activity and leakage

It is also advantageous that the enzyme activity (enzyme payload) after encapsulation be retained. The term "enzyme payload" or "enzyme activity" means the concentration in mass of active enzyme that is encapsulated in the delivery system. Typically, it is expressed as mass of active enzyme per total mass of the delivery system. The term "% enzyme activity after encapsulation" as recited herein refers to the encapsulation yield of active enzyme, or in other words, the assayed enzyme activity or enzyme payload after encapsulation relative to the starting enzyme activity or starting enzyme payload before encapsulation. The starting enzyme activity or starting enzyme payload before encapsulation is sometimes referred to as the theoretical payload. The enzyme activity of the encapsulated enzymes can be determined using techniques well known to those skilled in the art. The enzyme activity herein was determined by adding the enzyme delivery system (e.g., fiber mat having encapsulated enzyme) to a suitable liquid where all the enzyme is released or is made accessible for example with vigorous stirring (such as by relative centrifugal force of more than 4000) and then is assayed for enzyme according to techniques well known to those skilled in the art.

In some embodiments, percentage of active enzyme after encapsulation (i.e., encapsulation yield) is between and including any two of the following: 60, 65, 70, 75, 80, 85, 90, 95 to 100%.

In some embodiments, the percentage of active perhydrolase after encapsulation is from 60 to 100%. In another embodiment, the percentage of active perhydrolase after encapsulation is from 65 to 95%.

In another embodiment, the perhydrolase activity after encapsulation in the first water soluble fibers is comparable to perhydrolase activity of free enzyme in solution. In some embodiments, the perhydrolase activity after encapsulation does not decrease more than 10% compared to free perhydrolase, and in some embodiments, an increase may be seen.

The perhydrolase retains at least 70%, 85%, 90%, 91%, 92%, 93%, 95%, 95%, 96%, 97%, 98%, 99% or 100% of its initial activity (prior to encapsulation) after encapsulation. The perhydrolase after encapsulation retains 70 to 100% of its initial activity as free perhydrolase in solution. In some embodiments, perhydrolase after encapsulation retains between and optionally including any two of the following: 70%, 80%, 81%, 82%, 83%, 84%, 85%, 86%, 87%, 88%, 89%, 90%, 91%, 92%, 93%, 94%, 95%, 96%, 97%, 98%, 99% and 100% of its initial activity as free perhydrolase in solution. In another embodiment, the perhydrolase after encapsulation retains 90 to 100% of its initial activity as free perhydrolase in solution (encapsulation yield).

It is also desirable that the amount of enzyme that leaks out of the fibrous construct over time is minimized during its storage. It is also desirable that enzyme release only occurs with the desired trigger.

Enzyme leakage as used herein means the amount or fraction (which can be expressed as a weight percent) of the active enzyme released from a fibrous construct or portion of a fibrous construct such as a web. In the disclosure herein, enzyme leakage is usually expressed as a mass percent of active enzyme leaked relative to the original amount by mass of active enzyme encapsulated in the fibrous construct.

The enzyme leakage of a fibrous construct or part of a fibrous construct (such as a web) can be determined by taking known amounts of the fibrous construct which has a known amount of active enzyme (i.e., the initial enzyme payload) and dosing the fibrous construct into a known amount of aqueous composition. The aqueous composition can be stored under different conditions such as temperature and then analyzed at given time points for enzyme leakage into the aqueous composition. Leakage into the aqueous composition can be determined by techniques known to those skilled in the art. For example, enzyme leakage can be determined by measuring the enzyme activity of the resulting

supernatant that remains after centrifuging at moderate conditions (e.g., relative centrifugal force of about 1000) the aqueous composition containing the fibrous construct to separate particles from the bulk aqueous composition. The enzyme activity can be determined for example by using the ThermoScientific Coomasie Plus™ Bradford Assay Kit (ThermoScientific Product 23236). As previously mentioned, the analysis for enzyme leakage can be performed at given points in time and under different storage conditions of the aqueous composition.

For the fibrous construct of the present disclosure, enzyme leakage is stable over time. If leakage occurs, it occurs mostly in the first day then remains stable over the next 200 days. In some embodiments, the percent enzyme leakage at day 14 is the same as day 200. In some embodiments, the total enzyme leakage is 21% or less after 200 days when the fibrous construct is suspended in an aqueous solution comprising 35 wt% water or less based on the total weight of the aqueous solution and wherein the aqueous solution is from 20 to 30 degrees C. In another embodiment, the total enzyme leakage is 10% or less after 200 days, when the fibrous construct is suspended in an aqueous solution comprising 35 wt% water or less based on total weight of the aqueous solution and wherein the aqueous solution is from 20 to 30 degrees C. In yet another embodiment, the total enzyme leakage is 4% or less after 200 days when the fibrous construct is suspended in an aqueous solution comprising 35 wt% water or less based on total weight of the aqueous solution and wherein the aqueous solution is from 20 to 30 degrees C.

In some embodiments, the total enzyme leakage is 21% or less after 14 days when the fibrous construct is suspended in an aqueous solution comprising 35 wt% water or less based on the total weight of the aqueous solution and wherein the aqueous solution is at a temperature from 20 to 30 degrees C.

In some embodiments, the enzyme leakage is between and including any two of the following: 10, 11, 12, 12.2, 13, 14, 15, 20, 25, 30, 31, 32 and 35% (based on the initial enzyme payload) after 30 minutes when the fibrous construct is suspended in an aqueous solution comprising 70 wt% water or less based on total weight of the aqueous solution and wherein the aqueous solution is from 20 to 30 degrees C.

In some embodiments, the enzyme leakage is less than 32% (based on the initial enzyme payload), after 30 minutes when the fibrous construct is suspended in an aqueous solution comprising 70 wt% water or less based on total weight of the aqueous solution and wherein the aqueous solution is from 20 to 30 degrees C.

In some embodiments, the enzyme leakage is less than 13% (based on the initial enzyme payload), after 30 minutes when the fibrous construct is suspended in an aqueous solution comprising 70 wt% water or less based on total weight of the aqueous solution and wherein the aqueous solution is from 20 to 30 degrees C.

In some embodiments, the enzyme leakage is between and including any two of the following: 20, 25, 26, 27, 28, 29, 30, and 35% (based on the initial enzyme payload) after 30 minutes when the fibrous construct is suspended in an aqueous solution comprising 100 wt% water or less based on total weight of the aqueous solution and wherein the aqueous solution is from 20 to 30 degrees C.

In some embodiments, the enzyme leakage is less than 28% (based on the initial enzyme payload), after 30 minutes when the fibrous construct is suspended in an aqueous solution comprising 100 wt% water or less based on total weight of the aqueous solution and wherein the aqueous solution is from 20 to 30 degrees C.

In some embodiments, the enzyme leakage is less than 26% (based on the initial enzyme payload), after 30 minutes when the fibrous construct is suspended in an aqueous solution comprising 100 wt% water or less based on total weight of the aqueous solution and wherein the aqueous solution is from 20 to 30 degrees C.

The trigger to release the perhydrolase, the oxidizing agent (such as hydrogen peroxide) and optionally an encapsulated or absorbed ester substrate from the fiber will depend on the amount of water and the temperature of the aqueous solution. In some embodiments, an aqueous solution having greater than 90wt% water and accompanied by vigorous agitation (relative centrifugal force of greater than 2000) are the triggers to release the perhydrolase, the oxidizing agent (such as hydrogen peroxide) and optionally an encapsulated or absorbed ester substrate from the fibers.

In some embodiments, an aqueous solution having greater than 70% water and a temperature from 20 to 30 degrees C is the trigger. In yet another embodiment, an aqueous solution having 40 to 70% water and a temperature greater than 30 degrees C may be the trigger.

In some embodiments, the trigger can vary with desired end use application.

The fibrous construct may be stored in an aqueous solution (or a formulation such as a liquid detergent composition) wherein the amount of water is 70 wt% or less. Water can be used to dilute the aqueous solution so as to increase the amount of water to greater than 70 wt%, thereby triggering release of the enzyme from the fibers.

Ester substrate

The ester substrate is a perhydrolase substrate that contains an ester linkage. Esters comprising aliphatic and/or aromatic carboxylic acids and alcohols may be utilized as substrates with perhydrolase enzymes. In some embodiments, the ester source is an acetate ester. In some embodiments, the ester source is selected from one or more of propylene glycol diacetate, ethylene glycol diacetate, triacetin, ethyl acetate and tributyrin. In some embodiments, the ester source is selected from the esters of one or more of the following acids: formic acid, acetic acid, propionic acid,

butyric acid, valeric acid, caproic acid, caprylic acid, nonanoic acid, decanoic acid, dodecanoic acid, myristic acid, palmitic acid, stearic acid, and oleic acid. In some embodiments, the ester substrate is selected from diacetin, triacetin, ethyl acetate, ethyl lactate or mixtures thereof.

In one embodiment, the ester substrate is absorbed on to at least a portion of the first web or absorbed on to at least a portion of the second web or absorbed on to at least a portion of both the first web and the second web.

The first web or the second web can be submersed in liquid ester substrate or in a liquid solution of a solid ester substrate to incorporate the liquid ester substrate or the solid ester substrate dissolved in liquid solution into the web by absorption or absorptive encapsulation or imbibition. Absorbent capacity and absorbent rate are performance parameters to be accounted for in nonwoven webs. The absorbent capacity is mainly determined by the interstitial space between the fibers, the absorbing and swelling characteristics of the material and the resiliency of the web in the wet state. The absorbency rate is governed by the balance between the forces exerted by the capillaries and the frictional drag offered by the fiber surfaces. For non-swelling materials, these properties are largely controlled by the capillary sorption of fluid into the structure until saturation is reached (L.F. Fryer, B.S. Gupta, Determination of Pore Size Distribution in Fibrous Webs and its Impact on Absorbency, Proceedings of 1996 Nonwovens Confernce, 1996, 321-327). The polymer type of the fibers, hydrophilic or hydrophobic, influences the inherent absorbent properties of the web. A hydrophilic fiber provides the capacity to absorb liquid via fiber imbibitions, giving rise to fiber swelling. It also attracts and holds liquid external to the fiber, in the capillaries, and structure voids. On the other hand, a hydrophobic fiber has only the latter mechanism available to it normally (Gupta, B.S., and Smith, D. K., Nonwovens in Absorbent Materials, Textile Sci. and Technol. 2002,13, 349-388). Hence, the hydrophilicity, high surface area and high porosity of the web per mass and per volume of the web and the three dimensional porous network can be exploited to absorb or imbibe the liquid ester substrate or the solid ester substrate dissolved in liquid solution.

The absorption involves the submersion of a fiber matrix in the liquid ester substrate or in the solid ester substrate dissolved in liquid solution or the intimate physical contact of the liquid ester substrate or the solid ester substrate dissolved in liquid solution with the fiber matrix at desired fiber web:ester substrate ratio allowing the liquid to be absorbed within the 3D porous network, or partially within the individual fiber and/or adsorbed on the fiber surface.

In another embodiment, the fibrous construct additionally comprises a third web, the third web comprises a plurality of third water soluble fibers and wherein the ester substrate is absorbed on to at least a portion of the third web.

In another embodiment, the fibrous construct additionally comprises a third web, the third web comprises a plurality of third water soluble fibers and wherein the ester substrate is encapsulated in the third water soluble fibers.

In yet another embodiment, the ester substrate can be encapsulated with the perhydrolase as long as the enzyme survives the organic solvent that will dissolve the ester substrate and the polymer. In such embodiments, the organic solvent is selected from, but not limited, to acetone or chloroform.

To encapsulate the ester substrate (either solid or liquid) into a water soluble polymer resin, the ester substrate has to be dissolved with the water soluble polymer in a common solvent for solution spinning according to the procedure described in Example 1 and other procedures of solution spinning known in the art. For example, triacetin (CAS # 102-76-1), a liquid ester substrate that is practically insoluble in water (70 g/L at 25 °C), and polyvinylpyrrolidone, a water soluble polymer can both be dissolved in alcohols, including but not limited to, methanol, ethanol and isopropyl alcohol and also in chloroform and dicholoromethane.

The ester substrate, encapsulated or absorbed, is viable in generating peracetic acid in the enzymatic reaction involving triacetin as ester substrate and hydrogen peroxide as the oxidizing agent catalyzed by a perhydrolase enzyme.

In some embodiments, the ester substrate is present in an amount between and including any two of the following: 5, 10, 15, 20, 25, 30, 35, 40, 45, 50, 55, 60, 65, 70, 75, 84 and 85 wt% based on the total weight of the third web. In some embodiments, the ester substrate is present in an amount from 5 to 80 wt% based on the total weight of the third web. In another embodiment, the ester substrate is present in an amount from 5 to 60 wt% based on the total weight of the third web. In another embodiment, the ester substrate is present in an amount from 5 to 45 wt% based on the total weight of the third web. In another embodiment, the ester substrate is present in an amount from 5 to 30 wt% based on the total weight of the third web.

In another embodiment, the ester substrate is present in an amount from 5 to 60 wt% based on the total weight of the third web wherein the third web is electroblown. In another embodiment, the ester substrate is present in an amount between and including any two of the following: 5, 10, 15, 20, 25, 30, 35, 40, 45, 50, 55 and 60 wt% based on the total weight of the third web wherein the third web is electroblown. In yet another embodiment, the ester substrate is present in an amount from 5 to 30 wt% based on the total weight of the third web wherein the third web is electroblown.

In some embodiments, the third water soluble fibers having the ester substrate encapsulated have an average fiber diameter between and optionally including any two of the following numbers: 0.5, 1, 1.5 and 2 microns determined by scanning electron microscopy.

Oxidizing agent

The oxidizing agent is selected from peroxides, permanganate, chromate, dichromate, osmium tetroxide, perchlorate, potassium nitrate, perborate salts, percarbonate salts, nitrous oxide, silver oxide or mixtures thereof. In some embodiments, the oxidizing agent is hydrogen peroxide.

The oxidizing agent is encapsulated in the second water soluble fiber.

In some embodiments, the oxidizing agent is a hydrogen peroxide source that can spontaneously or enzymatically produce hydrogen peroxide.

In some embodiments, the hydrogen peroxide is complexed on to at least a portion of the second web. It is well known in the art that PVP and hydrogen peroxide form complexes upon contact as previously described herein (G. K. Surya Prakash, Anton Shakhmin, Kevin G. Glinton, Sneha Rao, Thomas Mathew, and George A. Olah. Green Chem. 2014, 16, 3616-3622.; E. F. Panarin, K. K. Kalnin'sh, and V. V. Azanova. Polym. Sci. Ser. A. 2007, 49 (3), 275-283; M. A. Zolfigol, G. Chehardoli, M. Shiri. Reac. Func. Polym. 2007, 67, 723-727; F. Haaf, A. Sanner, and F. Straub. Polym. J. 1985, 17 (1), 143-152.)

The hydrogen peroxide-polyvinylpyrrolidone (PVP) complexes are hydrogen-bonded complexes formed between the vinyl pyrrolidone (VP) side group of PVP and hydrogen peroxide. The vinyl pyrrolidone side group is a five member lactam ring with an amide carbonyl that is a strong hydrogen acceptor. On the other hand, hydrogen peroxide is a strong hydrogen donor, hence, it will form a stable complex with vinyl pyrrolidone. Molecularly, hydrogen peroxide complexes with VP as previously described.

PVP-hydrogen peroxide in powder or granule form is available as a commercial product (Peroxydone™, Ashland). The PVP- hydrogen peroxide complex of the present disclosure is in the form of a fiber mat, including nanofiber mats. An advantage of this fiber form is the higher surface area per volume or per mass in fibers relative to powders or films. A PVP-hydrogen peroxide complex can be obtained by preparing a PVP fiber mat (by electroblowing or other solution spinning techniques described earlier) and then performing the complexation with hydrogen peroxide in a solvent where the polymer is not soluble while the hydrogen peroxide is soluble. Since hydrogen peroxide as purchased is in aqueous solution and PVP fibers dissolve in aqueous solution, hydrogen peroxide may be extracted into the solvent. A number of suitable extraction solvents including diethyl ether and ethyl acetate were discovered to be useful. Hydrogen peroxide was extracted from aqueous solutions of hydrogen peroxide at room temperature by mixing desired amounts of 99 % pure ethyl acetate and 29 wt% aqueous $H_2O_2$ with gentle stirring (a relative centrifugal force of less than a 100).

The hydrogen peroxide complexed in PVP fibers are active and viable as a source of hydrogen peroxide in the perhydrolysis reaction involving triacetin as the ester substrate and hydrogen peroxide as the oxidizing agent catalyzed by perhydrolase.

In some embodiments, the hydrogen peroxide complexed in the second water soluble fibers is from 0.1 to 20 wt% based on the total weight of the second web. In some embodiments, the hydrogen peroxide complexed in the second water soluble fibers is from 5 to 15 wt% based on the total weight of the second web. In some embodiments, the hydrogen peroxide complexed in the second water soluble fibers is from 0.1 to 10.4 wt% based on the total weight of the second web.

In some embodiments, the hydrogen peroxide can be complexed on to a fragmented web. The fragmented web can be made from the second web, then the hydrogen peroxide is complexed on to at least a portion of the fragmented second web.

The encapsulated perhydrolase substantially does not react with the ester substrate. When the ester substrate, perhydrolase and oxidizing agent (such as hydrogen peroxide) are in contact, they react to produce a peracetic acid. Peracetic acid is used in cleaning, bleaching, disinfection or sterilization applications/compositions. Perhydrolase catalyzes perhydrolysis reaction to produce peracetic acid.

Fibrous construct

In one embodiment, the fibrous construct is made by simultaneously solution spinning the first web and the second and/or third web on to a collector. A polymer-enzyme solution and a polymer oxidizing agent solution are made. Each solution is extruded through a spinneret which is made up of an orifice or capillary nozzle. The solution comes out of the tip of the each nozzle, to form fibers, and the fibers are collected into a web on a collector. As fibers are formed, the solvent evaporates from the solution.

In one embodiment, the fibrous construct is made by simultaneously electro spinning the first web and the second and/or third web on to a grounded collector. A polymer-enzyme solution and a polymer oxidizing agent solution are made. Each solution is extruded through a spinneret which is made up of an orifice or capillary nozzle in which a high voltage is applied. Typically the voltage is in the range of 20-110 kV. As the solution comes out of the tip of the nozzle, to form fibers, and the fibers are collected as a web on a grounded collector. As fibers are formed, the solvent

evaporates from the solution.

In one embodiment, the fibrous construct is made by simultaneously electroblowing the first web and the second and/or third web on to a grounded collector. A polymer-enzyme solution and a polymer oxidizing agent solution are made. Each solution is extruded through a spinneret which is made up of an orifice or capillary nozzle in which a high voltage is applied. As the solution comes out of the tip of the nozzle, compressed air is blown directly toward the solution to form fibers, and the fibers are collected into a web on a grounded collector. As fibers are formed, the solvent evaporates from the solution. The voltage, the enclosure temperature, process airflow rates are operated or set in the range of 20-110 kV, room temperature to 60 °C and 0-20 scfm.

In another embodiment the fibrous construct is made as described in any of the embodiments above but instead of the fibers being spun simultaneously, the webs are made by sequential spinning. Both methods are well known and are not discussed in detail herein.

In embodiments wherein there is a third web, the third web is made in accordance with any method described above with the ester substrate encapsulated. Or, the third web is made in accordance with any method described above without the ester substrate and then the ester substrate is absorbed on at least a portion of the third web after the third web is made.

In some embodiments, the second web is made in accordance with any method described above then the hydrogen peroxide is complexed on to at least a portion of the second web.

The fibrous construct dissolves in a solution having 70 wt % water or greater based on the total weight percent of the solution. The perhydrolase and oxidizing agent (and optionally the ester substrate when the ester substrate is encapsulated) are released and will come in to contact with each other, reacting to produce peracetic acid.

In another embodiment, the fibrous construct is in the form of a woven web, fragmented woven web, a non-woven web, a fragmented non-woven web, individual fibers or combinations thereof.

Another embodiment of the present disclosure provides an aqueous composition comprising any of the fibrous constructs described herein wherein the aqueous composition comprises 95, 90, 80 or 70 wt% or less of water based on total weight of the aqueous composition. In other embodiments, the aqueous composition contains any of the fibrous constructs described herein and from 35 wt% to 70 wt% or from 40 wt% to 70 wt% water based on the total weight of the aqueous composition.

In one embodiment, a fibrous construct comprising:

a) an ester substrate;
b) a first web comprising a plurality of first water soluble fibers and a perhydrolase, the perhydrolase is encapsulated in the first water soluble fibers and is present in an amount from 0.1 to 40 wt% based on the total weight of the first web;
c) a second web comprising a plurality of second water soluble fibers and an oxidizing agent, where the oxidizing agent is encapsulated in the second water soluble fibers.

where the first water soluble fibers and the second water soluble fibers are solution spun water soluble fibers.

The fibrous construct may comprise:

a) an ester substrate;
b) a first web comprising a plurality of first water soluble fibers and a perhydrolase, wherein the perhydrolase is encapsulated in the first water soluble fibers and is present in an amount from 0.1 to 40 wt% based on the total weight of the first web;
c) a second web comprising a plurality of second water soluble fibers and hydrogen peroxide, where the hydrogen peroxide is complexed on to at least a portion of the second web;

where the second water soluble fibers are polyvinyl pyrrolidone or copolymers thereof; and
where the first water soluble fibers and the second water soluble fibers are solution spun water soluble fibers.

In another embodiment, the fibrous constructs as described further comprise in addition to the first and second webs previously described herein, a third web containing a plurality of third water soluble fibers, where the ester substrate is encapsulated in the third water soluble fibers or absorbed onto at least a portion of the third web.

In alternative embodiments, the ester substrate may be absorbed on to at least a portion of the first web or absorbed on to at least a portion of the second web or absorbed on to at least a portion of both the first web and the second web.

In some embodiments, the first, second and optional third water soluble fibers of any of the fibrous constructs described herein are independently selected from methylcellulose, hydroxypropyl methylcellulose, guar gum, alginate, polyvinyl pyrrolidone, polyethylene oxide, polyvinyl alcohol, pullulan, polyaspartic acid, polyacrylic acid, copolymers thereof or mixtures thereof. In other embodiments, the first, second and optional third water soluble fibers are independently selected from polyvinyl pyrrolidone, polyvinyl alcohol, pullulan or mixtures thereof. In yet other embodiments, the

first, second and optional third water soluble fibers have a crystallinity from 20 to 54%.

In some embodiments of the fibrous construct, the oxidizing agent that is encapsulated in the plurality of second water soluble fibers is at least selected from peroxides, permanganate, chromate, dichromate, osmium tetroxide, perchlorate, potassium nitrate, perborate salts, percarbonate salts, nitrous oxide, silver oxide or mixtures thereof. In other embodiments the oxidizing agent that is encapsulated at least includes hydrogen peroxide.

In other embodiments, the ester substrate that is included in the fiber construct includes at least an ester selected from diacetin, triacetin, ethyl acetate, ethyl lactate or mixtures thereof.

In yet other embodiments of the fiber construct described herein, the perhydrolase is encapsulated with an encapsulation efficiency of from 80 to 100%. The fibrous construct in other embodiments may encapsulate perhydrolase in the first web at an encapsulation yield of 90% to 100%.

The fibrous construct in accordance with any of the above embodiments, may have the first water soluble fibers, the second water soluble fibers and the third water soluble fibers made by electrospinning. In other embodiments, have the first water soluble fibers, the second water soluble fibers and the third water soluble fibers may be made by an electroblowing process.

In some embodiments, the fibrous construct dissolves in a solution having 70 wt % water or greater based on the total weight percent of the solution.

In other embodiments, the perhydrolase of the fibrous construct is provided in a composition that is substantially free of cells or cell debris.

Although methods and materials similar or equivalent to those described herein can be used in the practice or testing of the present invention, suitable methods and materials are described herein.

## EXAMPLES

The materials, methods, and examples herein are illustrative only and, except as specifically stated, are not intended to be limiting.

### Description of Polymer Resins

ELVANOL 80-18 is a 98 to 98.8 % hydrolysed copolymer of PVA (polyvinyl alcohol) and another monomer and is available from Kuraray Co., Ltd. ELVANOL 70-03 is a 98 to 98.8 % hydrolysed PVA (polyvinyl alcohol) and is also available from Kuraray Co., Ltd.

### Fiber Diameter Measurements

Fiber diameters of the solution spun fibers were measured as follows. Fiber samples were plasma-coated with ~1-3 nm of Osmium using an OPC-80T Osmium Plasma Coater and subsequently analyzed by SEM with a Hitachi SU3500 and Hitachi SU5000 Variable Pressure (VP) microscopes with a thermionic and Schottky-Field emission guns, respectively, operated under pressure in the range of 60-120 Pa at 5-10 kV (SU3500) or 1-5 kV (SU5000 VP). The average fiber diameter was determined by measuring the diameter from at least 100 fibers in each sample.

### Example 1

Example 1 demonstrates that enzyme encapsulated in fiber showed enzyme activity almost identical to the free enzyme and that the enzyme distribution in the fiber is highly uniform.

The polyvinyl alcohol polymer used was ELVANOL 80-18. One part of liquid perhydrolase variant concentrate (about 3.5 wt%) was added to nine parts of a 15 wt % aqueous ELVANOL 80-18 PVA solution to produce a polymer-enzyme solution. The perhydrolase enzyme-containing polyvinyl alcohol (PVA) fibers are prepared using electroblowing. The polymer-enzyme solution is extruded through a spinneret which is made up of an orifice or capillary nozzle in which a high voltage is applied. As the solution comes out of the tip of the nozzle, compressed air is blown directly toward the solution to form fibers, and the fibers are collected into a web on a grounded collector. As fibers are formed, the solvent evaporates from the solution. The voltage, the enclosure temperature, process air flow rates are operated or set respectively in the range of 20-110 kV, room temperature to 60 °C and 0-20 standard cubic feet per minute (scfm). This corresponded to a resulting fiber mat with 2.2 wt % encapsulated enzyme.

One part of liquid perhydrolase variant concentrate (about 3.5 wt%) was added to ninety parts of a 15 wt% aqueous ELVANOL 80-18 PVA solution and a fiber mat was produced in the same manner as described above. This corresponded to a resulting fiber mat with 0.2 wt % of encapsulated enzyme.

The fiber diameter of the enzyme encapsulated PVA fibers were analyzed according to the procedure previously described. The average fiber diameter was determined by measuring the diameter from at least 100 fibers in each

sample. Results are shown in Table 1.

**Table 1**

| | PVA fibers (0 wt% enzyme) | PVA fibers (0.2 wt% enzyme) | PVA fibers (2.2 wt% enzyme) |
|---|---|---|---|
| Ave. diameter | 359 ± 214 nm | 507 ± 269 nm | 585 ± 336 nm |

The distribution of the enzyme in the fiber mat was determined by directly measuring the protein content using the sodium dodecyl sulfate polyacrylamide gel electrophoresis (SDS-PAGE) protocol or the Laemmli method (Laemmli, U. K. (1970)) Cleavage of structural proteins during the assembly of the head of bacteriophage T4. Nature 227(5259): 680-685). Ten small sections (-0.4 in x ~0.4 in) were cut from different areas of the fiber mat which contained 2.2 wt% of encapsulated perhydrolase variant. The enzyme content of each of the 10 cut sections was measured by SDS-PAGE and compared to the known amount (theoretical amount) of enzyme used in the starting spinning solution. Table 2 provides the results of the SDS-PAGE analysis showing that the enzyme distribution in the fiber mat is highly uniform with an average of 0.86 ± 0.09 μg compared to the theoretical amount of 0.90 μg.

The encapsulation efficiency was determined and is a measure of the amount of enzyme encapsulated in the fiber mat relative to the amount of enzyme used in the starting solution for spinning as previously described. The average encapsulation efficiency of all the measured cut sections of the fiber mat is quite high close to 100% at 95 ± 10%, where the difference could be within statistical or random error, demonstrating that the method of encapsulation may be more efficient than conventional encapsulation methods like spray drying and fluidized bed processes. Results are shown in Table 2.

**Table 2**

| Sample | Enzyme Mass from SDS-PAGE (μg) | Theroretical Mass (μg) | Encapsulation Efficiency (%) |
|---|---|---|---|
| 1 | 0.89 | 0.90 | 99 |
| 2 | 1.06 | 0.90 | 118 |
| 3 | 0.92 | 0.90 | 102 |
| 4 | 0.87 | 0.90 | 97 |
| 5 | 0.86 | 0.90 | 96 |
| 6 | 0.76 | 0.90 | 84 |
| 7 | 0.81 | 0.90 | 90 |
| 8 | 0.75 | 0.90 | 83 |
| 9 | 0.88 | 0.90 | 98 |
| 10 | 0.78 | 0.90 | 87 |
| **Average** | **0.86** | **0.90** | **95** |
| **Std Dev** | **±0.09** | **0.90** | **± 10** |

The activity of the perhydrolase enzyme encapsulated in PVA fibers of the fiber mat containing 2.2 wt% of perhydrolase variant was determined by peracetic acid production in a buffered aqueous medium upon reaction of the fiber mat with triacetin and hydrogen peroxide. A new fiber mat composed of ELVANOL 80-18 with enzyme content of 2.2 wt % was made as described above. Cut electroblown fiber mats composed of sample size ranges from 25 $mm^2$ to 1 $cm^2$ with corresponding weights ranging from 1 to 80 mg were used. The reaction medium contained 4 mL buffer (100 mM sodium phosphate, pH 7.2) containing 150 - 200 mM triacetin and hydrogen peroxide (30 - 200 mM). A quantity of fiber mat equivalent to 80 μg/mL of perhydrolase enzyme was added to the reaction medium. The reactions were sampled at 0, 5, 15, 30, 60 and 105 minutes by transfer of 180 uL of the reaction solution to a vial containing 20 uL of 1.0 M phosphoric acid to terminate the enzyme reaction. The acid-quenched solutions were centrifuged (12,000 rpm, 5 min). The supernatant (0.010 - 0.050 mL) was transferred to a screw-capped glass HPLC vial containing 0.300 mL HPLC grade water for the subsequent modified Karst derivatization protocol (Pinkernell, U. Effkemann, S. Karst, U. Simultaneous HPLC determination of Peroxyacetic acid and hydrogen peroxide, 1997, Anal. Chem. 69 (17), 3623-3627). To initiate the first Karst derivatization reaction, 0.100 mL of 20 mM MTS (methyl-p-tolyl-sulfide) in acetonitrile was added using a positive displacement pipet. The vials were capped and the contents were gently mixed before a 10-minute incubation in the

dark at ca. 25°C. Subsequently, 0.400 mL acetonitrile and 0.100 mL 49 mM TPP (triphenylphosphine) in acetonitrile were added to each vial. The vials were vortexed to mix the contents and the vials were incubated in the dark for 30 minutes at *ca*. 25°C. An internal standard solution, 0.100 mL of 2.5 mM DEET (N,N-diethyl-m-toluamide) in acetonitrile was then added using a positive displacement pipet, the vials recapped and the contents were vigorously shaken to mix. The samples were evaluated by HPLC and analyzed for MTSO (methyl-tolyl-sulfoxide).

Two control reactions were also conducted to compare the rate and magnitude of perhydrolysis reaction with that of the fiber encapsulated perhydrolase. One control is the perhydrolysis reaction involving a PVA fiber mat of ELVANOL 80-18 without an encapsulated perhydrolase and the other control is the perhydrolysis reaction involving a free enzyme in solution. Table 3 summarizes the results of determining the activity of the enzyme perhydrolase in ELVANOL 80-18 with an enzyme content of 2.2 wt % by measuring the peracetic acid generation. Each measurement is usually an average of at least three replicates. The encapsulated enzyme in fiber showed enzyme activity almost identical to the free enzyme in solution as indicated by the generated peracetic acid in the perhydrolysis reaction which demonstrates that the encapsulation of the enzyme in polymeric fiber by electroblowing process does not have any deleterious effect on the enzyme and that the encapsulated enzyme is accessible when placed in aqueous solution. The fiber containing no enzyme showed quite limited peracetic acid production. Results are shown in Table 3.

**Table 3**

| **Time (min)** | **Peracetic Acid Generation (ppm)** | | |
|---|---|---|---|
| | **Free enzyme in Solution (control)** | **Enzyme encapsulated in Fiber** | **No Enzyme in Fiber (control)** |
| 0 | 0 | 0 | 0 |
| 5 | 6564 | 6160 | 135 |
| 15 | 6654 | 6986 | 292 |
| 30 | 6821 | 6347 | 439 |
| 60 | 6346 | 6365 | 494 |

**Example 2**

Example 2 demonstrates 0.17% and 1.7% perhydrolase encapsulated in pullulan fibers where enzyme activity is maintained. Perhydrolase enzyme-containing pullulan fibers were prepared using an electroblowing method as in Example 1. Pullulan is a water-soluble polysaccharide consisting of consecutive maltotriose units bound by $\alpha$-1,6 glucosidic linkages. White powder and odorless food grade pullulan purchased from Hayashibara Co., Ltd. of Japan was dissolved in water with stirring to prepare the pullulan solution. One part of liquid perhydrolase enzyme concentrate (about 3.5 wt%) was added to seven and a half parts of a 20 wt % aqueous pullulan solution. This corresponded to a resulting fiber mat with 1.7 wt % of encapsulated enzyme. In another, one part of liquid perhydrolase enzyme concentrate (about 3.5 wt%) was added to seventy five parts of a 15 wt% aqueous pullulan solution. This corresponded to a resulting fiber mat with 0.17 wt % of encapsulated enzyme. Fiber diameter of the pullulan fiber encapsulated enzymes were analyzed according to the method previously described. The average fiber diameter was determined by measuring the diameter from at least 100 fibers in each sample. Results are shown in Table 4.

**Table 4**

| **Pullulan fibers (0 wt% enzyme)** | **Pullulan fibers (0.17 wt% enzyme)** | **Pullulan fibers (1.7 wt% enzyme)** |
|---|---|---|
| ave diameter 944 $\pm$ 540 nm | ave diameter 833 $\pm$ 251 nm | ave diameter 417 $\pm$ 237 nm |

The activity of the perhydrolase enzyme in pullulan fibers was evaluated using the same procedure as in Example 1. Table 5 summarizes the results of determining the activity of the enzyme perhydrolase encapsulated in the pullulan fibers by measuring the peracetic acid generation. Sample A represents a 6.3 mg pullulan fiber mat cut containing 0.17 wt% of perhydrolase which is equivalent to a total of 0.011 mg of perhydrolase in the perhydrolysis reaction. Sample B represents a 2.7 mg of pullulan fiber mat cut containing 1.7 wt% of perhydrolase which is equivalent to a total of 0.046 mg of perhyrdolase in the perhydrolysis reaction. The data showed that the peracetic acid generation of both samples of pullulan-containing fibers was substantially much higher than the fiber containing no enzyme demonstrating the maintained enzyme activity. The peracetic acid generation increased with increase in enzyme content in the reaction

solution. Results are shown in Table 5.

**Table 5**

| Time (min) | Peracetic Acid Generation (ppm) | | |
|---|---|---|---|
| | No Enzyme in Fiber (control) | Sample A Enzyme Encapsulated in Pullulan Fiber (0.17 wt%) | Sample B Enzyme Encapsulated in Pullulan Fiber (1.7 wt%) |
| 0 | 0 ± 0 | 0 ± 0 | 0 ± 0 |
| 1 | 10 ± 3 | 74 ± 8 | 550 ± 25 |
| 5 | 23 ± 1 | 769 ± 209 | 1520 ± 13 |
| 15 | 39 ± 2 | 1070 ± 414 | 1971 ± 71 |
| 31 | 60 ± 2 | 1066 ± 329 | 1920 ± 13 |
| 60 | 103 ± 2 | 1185 ± 510 | 1924 ± 11 |

**Example 3**

Example 3 demonstrates that a liquid ester substrate, triacetin, can be absorbed and incorporated into the solution spun fiber web and that the absorbed triacetin in solution fiber web is viable in generating peracetic acid in the enzymatic reaction involving triacetin as ester substrate and hydrogen peroxide as the oxidizing agent catalyzed by a perhydrolase enzyme.

Three fiber webs to absorb triacetin were prepared: 1. Polyvinylpyrrolidone (PVP) fiber web without encapsulated enzyme, 2. ELVANOL 80-18 fiber web without encapsulated enzyme and 3. ELVANOL 80-18 fiber web with 2.2 wt% encapsulated perhydrolase enzyme. All three fiber webs were prepared using electroblowing as described in Example 1 from three different aqueous spinning solutions. All three aqueous spinning solutions were made in high purity water with a resistivity of 18.2 MΩ.cm and was obtained from an inline Millipore Synergy® UV water purification system. The PVP spinning solution consisted of 1 part of PVP (MW=1300 kDa, purchased from Sigma-Aldrich and used without further purification) added to 5.67 parts of high purity water, the mixture stirred vigorously at room temperature to obtain a clear solution with 15 wt% PVP. The ELVANOL 80-18 spinning solution consisted of 1 part of ELVANOL 80-18 added to 5.67 parts of high purity water the mixture stirred vigorously at room temperature to obtain a clear solution with 15 wt% ELVANOL 80-18. The ELVANOL 80-18 spinning solution with perhydrolase enzyme was prepared as described in Example 1.

The absorption of the ester substrate can be achieved by submersion of a fiber matrix in the liquid ester substrate or in the solid ester substrate dissolved in liquid solution or the intimate physical contact of the liquid ester substrate with the fiber matrix at desired fiber web:ester substrate ratio allowing the liquid to be absorbed within the 3D porous network, or partially within the individual fiber and adsorbed on the fiber surface. The liquid ester substrate (ex. triacetin) readily wetted the polyvinyl alcohol (PVA) and PVP nanofiber matrices with or without the encapsulated perhydrolase enzyme. Experiments showed the wetting of triacetin in the fiber web and the fiber morphology before and after the absorptive encapsulation can be seen from the SEM images that there is an increase in fiber diameter (almost doubled) with the presence of immobilized triacetin and that the open spaces between fibers in the matrix are covered.

The triacetin content after absorption by the fiber web with or without encapsulated enzyme ranges from 5 wt% to 84 wt % which is equivalent to 0.05 times to 5.25 times of absorbed triacetin, respectively, to the weight of the fiber web while the fiber matrices still feel and looked "dry".

The viability and accessibility of the triacetin absorbed in ELVANOL 80-18 fiber web containing 2.2 wt % perhydrolase enzyme were tested in the perhydrolysis enzymatic reaction described above by determining its ability to generate peracetic acid using the Karst HPLC assay as described in Example 1. The results are shown in Table 6 and demonstrate that the absorbed triacetin in fiber webs is accessible and viable in the enzymatic reaction and generate peracetic acid with time up to an hour. This also demonstrates that the encapsulated perhydrolase with the absorbed triacetin is active and viable in the enzymatic reaction and generate peracetic acid (PAA). The enzyme activity and PAA generation of the absorbed triacetin was also compared to neat liquid triacetin and they show close agreement demonstrating that the absorbed triacetin in the fiber matrix is as available and viable as the neat liquid triacetin.

**Table 6**

| Time (min) | Peracetic Acid Generation (ppm) | |
|---|---|---|
| | **Perhydrolase encapsulated in Fiber + Liquid Triacetin** | **Perhydrolase encapsulated in Fiber + Absorbed Triacetin** |
| 0 | 0 ± 0 | 0 ± 0 |
| 1 | 676 ± 352 | 526 ± 63 |
| 5 | 1315 ± 469 | 1315 ± 107 |
| 15 | 1541 ± 198 | 1525 ± 18 |
| 30 | 1510 ± 92 | 1487 ± 85 |
| 60 | 1407 ± 26 | 1448 ± 127 |

**Example 4**

Example 4 demonstrates that a liquid ester substrate, triacetin, can be encapsulated in solution spun fibers and that the encapsulated triacetin in the solution spun fibers is viable in generating peracetic acid (PAA) in the enzymatic reaction involving triacetin as an ester substrate and hydrogen peroxide as the oxidizing agent catalyzed by a perhydrolase enzyme.

The polyvinylpyrrolidone polymer (MW=1300 kDa), triacetin (>99% purity) and ethanol (> 99% purity) were purchased from Sigma Aldrich and were used without further purification and processing. In one preparation, a spinning solution A consisting of one part triacetin and 2.33 parts polyvinylpyrrolidone (PVP) polymer were added into ten parts of ethanol. In another preparation, a spinning solution B consisting of one part triacetin, 2.33 parts of polyvinylpyrrolidone were added into 12.67 parts of ethanol. The spinning solution mixtures A and B were stirred thoroughly until clear solutions were obtained. The solution mixtures were spun according to the procedure as described in Example 1.

Solution spun fibers were obtained and their fiber diameters were analyzed using scanning electron microscopy as described in Example 1. The average fiber diameter obtained from spinning solution B was 1.46 ± 0.57 μm. The viability and accessibility of the triacetin encapsulated in PVP fibers were tested in the perhydrolysis enzymatic reaction by determining its ability to generate peracetic acid using the Karst HPLC assay as described in Example 1. In this assay the source of the triacetin ester substrate in the enzymatic reaction is the encapsulated triacetin in PVP fibers, while the perhydrolase enzyme and $H_2O_2$ are both in the form of liquid solutions. The results are shown in Table 7 and they show that the encapsulated triacetin in fiber matrices is viable in the enzymatic reaction and generate peracetic acid with time up to an hour.

**Table 7**

| Time (min) | Peracetic acid generation (ppm) |
|---|---|
| 0 | 0 |
| 1 | 160 |
| 5 | 473 |
| 15 | 759 |
| 31 | 788 |
| 60 | 781 |

**Example 5**

Example 5 demonstrates that hydrogen peroxide can be complexed and immobilized in polyvinylpyrrolidone fibers and that the complexed and immobilized hydrogen peroxide is active and viable in participating in a reaction involving either a permanganate reaction or an enzymatic reaction involving triacetin and perhydrolase enzyme to generate peracetic acid.

It is well known in the art that PVP and hydrogen perxoide form complexes upon contact. The hydrogen peroxide-polyvinylpyrrolidone (PVP) complexes are hydrogen-bonded complexes formed between the vinyl pyrrolidone (VP) side

group of PVP and hydrogen peroxide. The vinylpyrrolidone side group is a five member lactam ring with an amide carbonyl that is a strong hydrogen acceptor. On the other hand, hydrogen peroxide is a strong hydrogen donor, hence, it will form a stable complex with vinyl pyrrolidone. Molecularly, hydrogen peroxide and VP can form complexes in a one to one (1:1) and one to two (1:2) ratio as seen in the reaction mechanism. This complexation with hydrogen peroxide then can be formed in copolymers of PVP where the vinyl pyrrolidone side group is present.

PVP-hydrogen peroxide in powder or granule form is available as a commercial product (Peroxydone™, Ashland) but in the present disclosure, the PVP-hydrogen peroxide complex will be in the form of a fiber mat, such as a nanofiber web. An advantage of this fiber form is the higher surface area per volume or per mass in fibers relative to powders or films. To prepare a PVP-hydrogen peroxide fiber mat, a PVP fiber mat was fabricated (by electroblowing or other solution spinning techniques described earlier) as described in Example 3 and then the complexation with hydrogen peroxide was performed in a solvent where the polymer is not soluble and the hydrogen peroxide is soluble. Since hydrogen peroxide as purchased is in aqueous solution and PVP fibers dissolve in aqueous solution, the hydrogen peroxide had to be extracted into the solvent. Suitable solvents include, but are not limited to, diethyl ether or ethyl acetate (EtoAc).

For this example, aqueous hydrogen peroxide (30 %, Sigma Aldrich) was extracted using ethyl acetate (>99%, Sigma Aldrich). A 10-ml round bottomed flask equipped with a magnetic stir bar was placed on a balance. The weight of the flask was tared, and 1 g of ethyl acetate was added via a pipette. The flask was placed on a magnetic stirring plate, and 1-ml of 30wt% of hydrogen peroxide was slowly added via a plastic pipette. The solution was allowed to stir at room temperature for 0.5-2 h. After the desired time, the solution was placed in a 5-ml scintillation vial and allowed to stand for 15 minutes for the immiscible layers to separate. The organic layer was carefully removed via a micropipette.

Five (5) solutions are usually prepared for safety reasons. The organic layers are combined, and kept cold in a chemical refrigerator in between uses. The hydrogen peroxide concentration after extraction was evaluated via $KMnO_4$ titration which is known in the art and via Karst HPLC (Pinkernell, U.; Effkemann, S.; Karst, U. Simultaneous HPLC determination of peroxyacetic acid and hydrogen peroxide Anal. Chem. 69 3623-3627 (1997)) and the two assay procedures agreed well. The results are shown in Table 8.

**Table 8**

| Concentration of Hydrogen Peroxide in Ethyl Acetate After Extraction | | |
|---|---|---|
| Sample | $H_2O_2$ Concentration (wt%) | |
| | KMnO4 Titration | Karst HPLC |
| $H_2O_2$ extracted in Ethyl Acetate | 10.2 | 9.5 |

Hydrogen peroxide complexation with PVP fibers was conducted by submersing the PVP fiber mat in the hydrogen peroxide-ethyl acetate solution. A 20-ml glass jar was charged with 10-ml of ethyl acetate. The jar was then placed on an analytical balance, and the desired amount of freshly prepared hydrogen peroxide-ethyl acetate was added according to Table 9 below.

**Table 9: Preparation of PVP-$H_2O_2$ complexes in fibers**

| PVP fiber (9) | $H_2O_2$-EtOAc (9) | EtOAc (ml) | Yield (g) |
|---|---|---|---|
| 0.1517 | 1.7311 | 5 | 0.1521 |
| 0.1680 | 2.1286 | 10 | 0.1787 |
| 0.1590 | 1.7433 | 5 | 0.1719 |
| 0.1524 | 1.6355 | 10 | 0.1754 |
| 0.1528 | 1.2588 | 10 | 0.1745 |

The hydrogen peroxide-ethyl acetate solution was gently swirled before carefully adding the weighed PVP fibers (2 x 2 cm) using stainless steel tweezers. The fibers shrunk immediately after being placed in the hydrogen peroxide-ethyl acetate solution. The fibers were allowed to stand for 0.5 -1 h in the solution. After the desired time, the supernatant was decanted and kept for analysis. The fibers were rinsed with ethyl acetate before placing in a vacuum oven for drying (1-2 h) at ambient temperature. After the complexation reaction of PVP nanofibers and hydrogen peroxide in ethyl acetate, the resulting fiber mat decreased or shrunk in size, however, the fiber morphology as imaged by scanning electron microscopy (SEM) was still maintained. The PVP-hydrogen peroxide nanofiber matrices were less soluble in

water than the precursor PVP nanofibers.

The hydrogen peroxide concentration in the fiber was evaluated via $KMnO_4$ titration which is known in the art and via Karst HPLC (Pinkernell, U.; Effkemann, S.; Karst, U. Simultaneous HPLC determination of peroxyacetic acid and hydrogen peroxide Anal. Chem. 69 3623-3627 (1997)) and the two assay procedures agreed well. The results are shown in Table 10.

**Table 10**

| Concentration of $H_2O_2$ in PVP fiber after 1 hour of submersion | | |
|---|---|---|
| Sample | $H_2O_2$ Concentration (wt%) | |
| | KMnO4 Titration | Karst HPLC |
| PVP fiber-$H_2O_2$ complex | 9.1 | 10.4 |

**Example 6**

Example 6 demonstrates that the hydrogen peroxide complexed in PVP fibers are active and viable as a source of hydrogen peroxide in the perhydrolysis reaction involving triacetin as the ester substrate and hydrogen peroxide as the oxidizing agent catalyzed by perhydrolase.

Dried hydrogen peroxide-PVP fiber mats with an average hydrogen peroxide concentration of 9.8 wt% prepared in Example 5 were used as a source of $H_2O_2$ in the perhydrolysis reaction and the peracetic acid generation was measured according to the Karst assay as described in Example 1. Two different levels of hydrogen peroxide concentrations (18.0 mM and 29.5 mM) from the hydrogen peroxide-PVP fiber mats were utilized equivalent to 25.0 mg and 41.0 mg mass of hydrogen peroxide-PVP fiber mats, respectively. The results of peracetic acid generation are shown in Table 11 demonstrating that the complexed hydrogen peroxide in PVP fiber is active and viable.

**Table 11**

| Peracetic Acid Generation by $H_2O_2$ complexed in PVP fibers | | |
|---|---|---|
| Time (min) | Peracetic Acid Generation (ppm) | |
| | 18.0 mM from $H_2O_2$-PVP fiber | 29.5 mM from $H_2O_2$-PVP fiber |
| 0 | $0 \pm 0$ | $0 \pm 0$ |
| 1 | $522 \pm 124$ | $1097.5 \pm 131.9$ |
| 5 | $887.5 \pm 218.5$ | $1344.5 \pm 13.8$ |
| 15 | $767 \pm 203.6$ | $1168.5 \pm 8.8$ |
| 30 | $632 \pm 169.7$ | $983.5 \pm 1.8$ |
| 60 | $482.5 \pm 143.5$ | $784 \pm 4.9$ |

**Example 7**

Example 7 demonstrates a quantitative measurement of PVA solubility that was performed using the spectrophotometric method described in the published journal article "Simple spectrophotometric method for determination of polyvinyl alcohol in different types of wastewater, L. Procházková, Y. Rodríguez-Muñoz, J. Procházka, J. Wannera, 2014, Intern. J. Environ. Anal. Chem., 94, 399-410" based on complexation with iodine according to the Pritchard method that has been previously described in earlier publications including the following: I.F. Aleksandrovich and L.N. Lyubimova, Fibre Chem. 24, 156 (1993); D.P. Joshi, Y.L. Lan-Chun-Fung and J.G. Pritchard, Anal. Chim. Acta. 104, 153 (1979); Y. Morishima, K. Fujisawa and S. Nozakura, Polym. J. 10, 281 (1978); J.G. Pritchard and D.A. Akintola, Talanta. 19, 877 (1972).

Quantitative PVA Solubility Analysis

Electroblown fiber mat samples from ELVANOL 80-18 were prepared as described in Example 1. These fiber mats were vacuum dried overnight, were weighed, and placed in 20-ml scintillation vials. Propylene glycol was purchased from Sigma-Aldrich and high purity water with a resistivity of 18.2 MΩ.cm was obtained from an inline Millipore Synergy®

UV water purification system. The water-propylene glycol mixtures at varying water content ranging from 0, 15, 30, 40, 50, 70 and 100 % were added to the vials to give a final solid concentration of 3.5 wt% and 0.5 wt% in the mixture and were stirred for at least 24 hours. The mixture was centrifuged and an aliquot is taken for PVA determination.

PVA content was measured via UV-Vis spectrophotometric method based on the above journal article using boric acid and iodine as follows: 500 μl of the aliquot was added to a 5-ml Eppendorf tube. 1500 μl of boric acid (0.04 g/ml) was added, and the solution was vortexed. 2000 μl distilled Millipore water was added to bring the final volume to 4000 μl. The solution was vortexed, and 1000 μl iodine solution was added, and vortexed. The solution was incubated for 15 minutes and the absorbance was measured at 590 nm. Samples were prepared in duplicate.

Results of the measurement are shown in Table 12. The overall solubility is expressed in two ways: in wt % of initial solid, meaning the percentage amount of the initial solid that was placed in the solvent mixture that dissolved, and in mg/ml, meaning the amount of solid in mg that dissolved in every ml of solvent. As water content is increased in the mixtures up to 50 %, PVA solubility increased from 0 to 19 wt % or equivalent to 0 to 6.6 mg /ml for the 3.5 wt % solids concentration level in mixture and from 0 to 21 wt % or equivalent to 0 to 1.0 mg/ml for the 0.5 wt % solids concentration level in mixture. These are interesting results as they indicate that though the fiber mat disintegrated, shrunk and/or disappeared as seen from the naked eye, not all are theoretically or quantitatively dissolved. Likely, the undissolved PVA that disappeared are too small to be seen by the naked eye.

The solubility behavior in water of the PVA fiber mats in comparison to PVA powders as obtained from the manufacturer was determined by visual inspection. ELVANOL 70-03 and ELVANOL 80-18 both as obtained from the manufacturer do not dissolve in water at room and cold temperature.

To dissolve ELVANOL 70-03 and ELVANOL 80-18, the water solvent as recommended is to be heated to at least 90 °C before PVA is added, and then the PVA-water mixture is recommended to be continuously heated with agitation. When transformed into fibers, it was surprisingly discovered that there was rapid fragmentation and eventual disappearance of the ELVANOL 70-03 and ELVANOL 80-18 fibers in water.

The solubility behavior of the fibers was determined by placing in a 150 ml beaker approximately 1.2 in x 1.2 in of square fiber section in high purity water with a resistivity of 18.2 MΩ-cm obtained from an in-line Millipore Synergy® UV water purification system at a PVA concentration of 0.1 wt%. The water was cooled in a recirculating bath (Thermo Electron Corporation, Neslar Merlin M25) filled with 50:50 water:ethylene glycol in a 250 mL-jacketed reaction kettle to the desired temperature and monitored with a 80PK-1 temperature probe connected to a digital readout display (Fluke 52II). The water cooled at the desired temperature was decanted and used in the visual solubility measurements. The temperature of the water-PVA mixture was monitored using an alcohol thermometer accurate to ±1 °C.

The fiber mat placed in water disappeared visually within 2 minutes at room temperature (~25°C), between 2.5-5 min at 15°C, between 3-7 min at 10°C, between 5-10 min at 5°C and between 10-20 min at close to 0°C.

Table 12 shows quantitative solubility measurements of ELVANOL 80-18 fiber mats and powder as received from Kuraray Co., Ltd using spectrophotometric method.

**Table 12**

| Sample | Propylene Glycol-Water Mixture Composition (%) | Overall Solubility (wt% based on wt of initial solid) | Overall Solubility (mg/ml) |
|---|---|---|---|
| **ELVANOL 80-18 fiber mat in 3.5 wt% solids in mixture (35 mg/ml)** | | | |
| *1* | 0% Water | 0.0% | 0.0 |
| *2* | 15% Water | 0.1% | 0.0 |
| *3* | 30% Water | 11.6% | 4.1 |
| *4* | 40% Water | 15.0% | 5.2 |
| *5* | 50% Water | 18.8% | 6.6 |
| *6* | 70% Water | 18.6% | 6.5 |
| *7* | 100% Water | 19.7% | 6.9 |
| **ELVANOL 80-18 fiber mat in 0.5 wt% solids in mixture (5 mg/ml)** | | | |
| *1* | 0% Water | 0.1% | 0.0 |
| *2* | 15% Water | 0.1% | 0.0 |
| *3* | 30% Water | 13.3% | 0.7 |
| *4* | 40% Water | 19.3% | 1.0 |

(continued)

| ELVANOL 80-18 fiber mat in 0.5 wt% solids in mixture (5 mg/ml) | | | |
|---|---|---|---|
| **5** | 50% Water | 21.2% | 1.1 |
| **6** | 70% Water | 21.2% | 1.1 |
| **7** | 100% Water | 19.3% | 1.0 |
| **ELVANOL 80-18 Powder (as received) in 3.5 wt% solids in mixture (35 mg/ml)** | | | |
| **1** | 0% Water | 0.0% | 0.0 |
| **2** | 15% Water | 0.1% | 0.0 |
| **3** | 30% Water | 0.1% | 0.0 |
| **4** | 40% Water | 0.2% | 0.1 |
| **5** | 50% Water | 0.2% | 0.1 |
| **6** | 70% Water | 0.4% | 0.1 |
| **7** | 100% Water | 0.8% | 0.3 |

Effect of Fiber Crystallinity and Size on Solubility

The release of encapsulated actives such as enzyme can be controlled by controlling the PVA polymer solubility through such parameters as the PVA polymer crystallinity and fiber size.

The crystallinity of the fiber samples was measured using dynamic scanning calorimetry (DSC). To optimize the temperature and time of the drying process in the DSC cell, few experiments were performed on the samples at 75°C and 100°C with timing of 5 and 10 minutes. The optimal temperature and time for drying that was selected were 100°C and 10 minutes.

To measure the enthalpy of fusion of the dried PVA powder and fiber samples, DSC experiments were performed from 20 degrees C to 100 degrees C at 5°C/min. Samples were then held for 10 minutes at 100 degrees C before being cooled to 0 degrees C at 10°C/min. Experiments were then continued on the dried samples in one heat temperature profile from 0 degrees C to 250 degrees C at10°C/min in $N_2$ atmosphere using a Q1000 DSC from TA Instruments.

Table 13 demonstrates the varying solubility of PVA fiber mats as a function of crystallinity at constant fiber diameter. Solubility decreases as fiber crytallinity increases. To ensure constant fiber diameter when measuring crystallinity, samples from the same fiber mat were annealed at 120 degrees C and 150 degrees C for an hour and crystallinity measurements of the annealed fiber mats were compared a reference unannealed fiber mat sample.

**Table 13**

| Sample | Glass Transition Temperature | Average Fiber Size | Initial Crystallinity | $H_2O$ Solubility | $H_2O$ Solubility |
|---|---|---|---|---|---|
| | **Tg (°C)** | (μm) | (%) | (wt % of initial solid) | (mg/ml) |
| ELVANOL 70-03 fibers | 80.4 | 0.278 ± 0.199 | 34.7% | 22.5 | 7.9 |
| ELVANOL 70-03 fibers-annealed @120°C | 81.0 | 0.278 ± 0.199 | 48.8% | 9.8 | 3.4 |
| ELVANOL 70-03 fibers-annealed @150° | 81.4 | 0.278 ± 0.199 | 54.2 | 1.5 | 0.5 |

Table 14 demonstrates the effect of solubility of PVA fibers as a function of fiber size at constant crystallinity. Solubility decreased as fiber sizes increased. The 20 micron sized fibers with similar crystallinity were prepared using wet spinning as described by Sakurada, I. Polyvinyl alcohol fibers. 1985, Marcel Dekker, Inc. NY.

Table 14

| Sample | Glass Transition Temperature | Initial Crystallinity | Average Fiber Size | $H_2O$ Solubility | $H_2O$ Solubility |
|---|---|---|---|---|---|
| | **Tg (°C)** | (%) | (μm) | (wt % of initial solid) | (mg/ml) |
| ELVANOL 80-18 fibers | 78.3 | 28.1% | 0.901 ± 0.8 | 18.8 | 6.6 |
| ELVANOL 80-18 fibers | 76.0 | 22.0% | 1.652 ± 0.7 | 16.3 | 5.7 |
| ELVANOL 80-18 wet-spun fibers | 79.9 | 31.6% | 20.0 ± 2.0 | 11.0 | 3.0 |

Note that not all of the activities described above in the general description or the examples are required, that a portion of a specific activity may not be required, and that further activities may be performed in addition to those described. Still further, the order in which each of the activities are listed are not necessarily the order in which they are performed. After reading this specification, skilled artisans will be capable of determining what activities can be used for their specific needs or desires.

In the foregoing specification, the invention has been described with reference to specific embodiments. However, one of ordinary skill in the art appreciates that various modifications and changes can be made without departing from the scope of the invention as set forth in the claims below.
Accordingly, the specification is to be regarded in an illustrative rather than a restrictive sense and all such modifications are intended to be included within the scope of the invention.

Benefits, other advantages, and solutions to problems have been described above with regard to specific embodiments. However, the benefits, advantages, solutions to problems, and any element(s) that may cause any benefit, advantage, or solution to occur or become more pronounced are not to be construed as a critical, required, or essential feature or element of any or all the claims.

## Claims

1. A fibrous construct comprising:

   a) an ester substrate;
   b) a first web comprising a plurality of first water soluble fibers and a perhydrolase, wherein the perhydrolase is encapsulated in the first water soluble fibers and is present in an amount from 0.1 to 40 wt% based on the total weight of the first web;
   c) a second web comprising a plurality of second water soluble fibers and an oxidizing agent, wherein the oxidizing agent is encapsulated in the second water soluble fibers; and

   wherein the first water soluble fibers and the second water soluble fibers are solution spun water soluble fibers.

2. The fibrous construct in accordance with claim 1, further comprising a third web, the third web comprising a plurality of third water soluble fibers and wherein the ester substrate is encapsulated in the third water soluble fibers.

3. The fibrous construct in accordance with claim 1, further comprising a third web, the third web comprising a plurality of third water soluble fibers and wherein the ester substrate is absorbed on to at least a portion of the third web.

4. The fibrous construct in accordance with claims 2 or 3, wherein the first water soluble fibers, the second water soluble fibers and the third water soluble fibers are independently selected from methylcellulose, hydroxypropyl methylcellulose, guar gum, alginate, polyvinyl pyrrolidone, polyethylene oxide, polyvinyl alcohol, pullulan, polyaspartic acid, polylactic acid, polyacrylic acid, copolymers thereof or mixtures thereof.

5. The fibrous construct in accordance with any one of claims 1 to 4, wherein the ester substrate is absorbed on to at least a portion of the first web or absorbed on to at least a portion of the second web or absorbed on to at least a portion of both the first web and the second web.

6. The fibrous construct in accordance with any one of claims 1 to 5, wherein the first water soluble fibers and the

second water soluble fibers are independently selected from methylcellulose, hydroxypropyl methylcellulose, guar gum, alginate, polyvinyl pyrrolidone, polyethylene oxide, polyvinyl alcohol, pullulan, polyaspartic acid, polylactic acid, polyacrylic acid, copolymers thereof or mixtures thereof.

**7.** The fibrous construct in accordance with any one of claims 1 to 6, wherein the first water soluble fibers and the second water soluble fibers have a crystallinity from 20 to 54%.

**8.** The fibrous construct in accordance with any one of claims 1 to 7, wherein the oxidizing agent is selected from peroxides, permanganate, chromate, dichromate, osmium tetroxide, perchlorate, potassium nitrate, perborate salts, percarbonate salts, nitrous oxide, silver oxide or mixtures thereof.

**9.** The fibrous construct in accordance with claims 1, 2 or 3, said construct comprising:

a) an ester substrate;
b) a first web comprising a plurality of first water soluble fibers and a perhydrolase, wherein the perhydrolase is encapsulated in the first water soluble fibers and is present in an amount from 0.1 to 40 wt% based on the total weight of the first web;
c) a second web comprising a plurality of second water soluble fibers and hydrogen peroxide, wherein the hydrogen peroxide is complexed on to at least a portion of the second web;

wherein the second water soluble fibers are polyvinyl pyrrolidone or copolymers thereof; and
wherein the first water soluble fibers and the second water soluble fibers are solution spun water soluble fibers.

**10.** The fibrous construct in accordance with claim 9, wherein the first water soluble fibers and the third water soluble fibers are independently selected from methylcellulose, hydroxypropyl methylcellulose, guar gum, alginate, polyvinyl pyrrolidone, polyethylene oxide, polyvinyl alcohol, pullulan, polyaspartic acid, polylactic acid, polyacrylic acid, copolymers thereof or mixtures thereof.

**11.** The fibrous construct in accordance with claim 9, wherein the ester substrate is absorbed on to at least a portion of the first web.

**12.** The fibrous construct in accordance with claim 9, wherein the first water soluble fibers are selected from methylcellulose, hydroxypropyl methylcellulose, guar gum, alginate, polyvinyl pyrrolidone, polyethylene oxide, polyvinyl alcohol, pullulan, polyaspartic acid, polylactic acid, polyacrylic acid, copolymers thereof or mixtures thereof.

**13.** The fibrous construct in accordance with any of the preceding claims, wherein the ester substrate is selected from diacetin, triacetin, ethyl acetate, ethyl lactate or mixtures thereof.

**14.** The fibrous construct in accordance with any of the preceding claims wherein encapsulation efficiency of the perhydrolase is from 80 to 100%.

**15.** The fibrous construct in accordance with any of the preceding claims wherein; (i) the fibrous construct dissolves in a solution having 70 wt % water or greater based on the total weight percent of the solution; and/or (ii) the perhydrolase is provided in a composition that is substantially free of cells or cell debris; and/or (iii) the fibrous construct is in the form of a woven web, fragmented woven web, a non-woven web, a fragmented non-woven web, individual fibers or combinations thereof.

## Patentansprüche

**1.** Fasriges Konstrukt umfassend:

a) ein Estersubstrat;
b) eine erste Bahn umfassend eine Mehrzahl erster wasserlöslicher Fasern und eine Perhydrolase, wobei die Perhydrolase in den ersten wasserlöslichen Fasern eingekapselt ist und in einer Menge von 0,1 bis 40 Gew.-%, auf das Gesamtgewicht der ersten Bahn bezogen, vorliegt;
c) eine zweite Bahn umfassend eine Mehrzahl zweiter wasserlöslicher Fasern und ein Oxidationsmittel, wobei das Oxidationsmittel in den zweiten wasserlöslichen Fasern eingekapselt ist; und

wobei die ersten wasserlöslichen Fasern und die zweiten wasserlöslichen Fasern lösungsgesponnene, wasserlösliche Fasern sind.

**2.** Fasriges Konstrukt nach Anspruch 1, ferner eine dritte Bahn umfassend, wobei die dritte Bahn eine Mehrzahl dritter wasserlöslicher Fasern umfasst und wobei das Estersubstrat in den dritten wasserlöslichen Fasern eingekapselt ist.

**3.** Fasriges Konstrukt nach Anspruch 1, ferner eine dritte Bahn umfassend, wobei die dritte Bahn eine Mehrzahl dritter wasserlöslicher Fasern umfasst und wobei das Estersubstrat auf mindestens einen Teil der dritten Bahn absorbiert ist.

**4.** Fasriges Konstrukt nach den Ansprüchen 2 oder 3, wobei die ersten wasserlöslichen Fasern, die zweiten wasserlöslichen Fasern und die dritten wasserlöslichen Fasern unabhängig unter Methylcellulose, Hydroxypropylmethylcellulose, Guargummi, Alginat, Polyvinylpyrrolidon, Polyethylenoxid, Polyvinylalkohol, Pullulan, Polyasparaginsäure, Polymilchsäure, Polyacrylsäure, Copolymeren davon oder Mischungen davon ausgewählt sind.

**5.** Fasriges Konstrukt nach einem der Ansprüche 1 bis 4, wobei das Estersubstrat auf mindestens einen Teil der ersten Bahn absorbiert oder auf mindestens einen Teil der zweiten Bahn absorbiert oder auf mindestens einen Teil sowohl der ersten Bahn als auch der zweiten Bahn absorbiert ist.

**6.** Fasriges Konstrukt nach einem der Ansprüche 1 bis 5, wobei die ersten wasserlöslichen Fasern und die zweiten wasserlöslichen Fasern unabhängig unter Methylcellulose, Hydroxypropylmethylcellulose, Guargummi, Alginat, Polyvinylpyrrolidon, Polyethylenoxid, Polyvinylalkohol, Pullulan, Polyasparaginsäure, Polymilchsäure, Polyacrylsäure, Copolymeren davon oder Mischungen davon ausgewählt sind.

**7.** Fasriges Konstrukt nach einem der Ansprüche 1 bis 6, wobei die ersten wasserlöslichen Fasern und die zweiten wasserlöslichen Fasern eine Kristallinität von 20 bis 54 % aufweisen.

**8.** Fasriges Konstrukt nach einem der Ansprüche 1 bis 7, wobei das Oxidationsmittel unter Peroxiden, Permanganat, Chromat, Dichromat, Osmiumtetroxid, Perchlorat, Kaliumnitrat, Perboratsalzen, Percarbonatsalzen, Distickstoffoxid, Silberoxid oder Mischungen davon ausgewählt ist.

**9.** Fasriges Konstrukt nach den Ansprüchen 1, 2 oder 3, wobei das Konstrukt Folgendes umfasst:

a) ein Estersubstrat;
b) eine erste Bahn umfassend eine Mehrzahl erster wasserlöslicher Fasern und eine Perhydrolase, wobei die Perhydrolase in den ersten wasserlöslichen Fasern eingekapselt ist und in einer Menge von 0,1 bis 40 Gew.-%, auf das Gesamtgewicht der ersten Bahn bezogen, vorliegt;
c) eine zweite Bahn umfassend eine Mehrzahl zweiter wasserlöslicher Fasern und Wasserstoffperoxid, wobei das Wasserstoffperoxid auf mindestens einen Teil der zweiten Bahn komplexiert ist.

wobei die zweiten wasserlöslichen Fasern Polyvinylpyrrolidon oder Copolymere davon sind; und
wobei die ersten wasserlöslichen Fasern und die zweiten wasserlöslichen Fasern lösungsgesponnene, wasserlösliche Fasern sind.

**10.** Fasriges Konstrukt nach Anspruch 9, wobei die ersten wasserlöslichen Fasern und die dritten wasserlöslichen Fasern unabhängig ausgewählt sind unter Methylcellulose, Hydroxypropylmethylcellulose, Guargummi, Alginat, Polyvinylpyrrolidon, Polyethylenoxid, Polyvinylalkohol, Pullulan, Polyasparaginsäure, Polymilchsäure, Polyacrylsäure, Copolymeren davon oder Mischungen davon.

**11.** Fasriges Konstrukt nach Anspruch 9, wobei das Estersubstrat auf mindestens einen Teil der ersten Bahn absorbiert ist.

**12.** Fasriges Konstrukt nach Anspruch 9, wobei die ersten wasserlöslichen Fasern unter Methylcellulose, Hydroxypropylmethylcellulose, Guargummi, Alginat, Polyvinylpyrrolidon, Polyethylenoxid, Polyvinylalkohol, Pullulan, Polyasparaginsäure, Polymilchsäure, Polyacrylsäure, Copolymeren davon oder Mischungen davon ausgewählt sind.

**13.** Fasriges Konstrukt nach einem der vorhergehenden Ansprüche, wobei das Estersubstrat unter Diacetin, Triacetin, Ethylacetat, Ethyllactat oder Mischungen davon ausgewählt ist.

**14.** Faserkonstrukt nach einem der vorhergehenden Ansprüche, wobei die Einkapselungseffizienz der Perhydrolase 80 bis 100 % beträgt.

**15.** Fasriges Konstrukt nach einem der vorhergehenden Ansprüche, wobei: (i) das fasrige Konstrukt in einer Lösung gelöst ist, die 70 Gew.-% Wasser oder mehr, auf den Gesamtgewichtprozentsatz der Lösung bezogen, aufweist; und/oder (ii) die Perhydrolase in einer Zusammensetzung bereitgestellt ist, die im Wesentlichen frei von Zellen oder Zelltrümmern ist; und/oder (iii) das fasrige Konstrukt sich in Form einer gewobenen Bahn, fragmentierten gewobenen Bahn, einer Vliesstoffbahn, einer fragmentierten Vliesstoffbahn, einzelner Fasern oder Kombinationen davon befindet.

**Revendications**

**1.** Construction fibreuse comprenant:

a) un substrat ester;
b) un premier voile comprenant une pluralité de premières fibres solubles dans l'eau et une perhydrolase, où la perhydrolase est encapsulée dans les premières fibres solubles dans l'eau et est présente en une quantité de 0,1 à 40 % en pds sur la base du poids total du premier voile;
c) un second voile comprenant une pluralité de secondes fibres solubles dans l'eau et un agent oxydant, l'agent oxydant étant encapsulé dans les secondes fibres solubles dans l'eau; et

où les premières fibres solubles dans l'eau et les secondes fibres solubles dans l'eau sont des fibres solubles dans l'eau filées en solution.

**2.** Construction fibreuse selon la revendication 1, comprenant en outre un troisième voile, le troisième voile comprenant une pluralité de troisièmes fibres solubles dans l'eau et le substrat ester étant encapsulé dans les troisièmes fibres solubles dans l'eau.

**3.** Construction fibreuse selon la revendication 1, comprenant en outre un troisième voile, le troisième voile comprenant une pluralité de troisièmes fibres solubles dans l'eau et le substrat ester étant absorbé sur au moins une partie du troisième voile.

**4.** Construction fibreuse selon les revendications 2 ou 3, les premières fibres solubles dans l'eau, les secondes fibres solubles dans l'eau et les troisièmes fibres solubles dans l'eau étant indépendamment sélectionnées parmi la méthylcellulose, l'hydroxypropyl méthylcellulose, la gomme de guar, l'alginate, la polyvinyl pyrrolidone, le poly(oxyde d'éthylène), le poly(alcool de vinyle), le pullulane, le poly(acide aspartique), le poly(acide lactique), le poly(acide acrylique), leurs copolymères ou leurs mélanges.

**5.** Construction fibreuse selon l'une quelconque des revendications 1 à 4, dans laquelle le substrat ester est absorbé sur au moins une partie du premier voile ou absorbé sur au moins une partie du second voile ou absorbé sur au moins une partie à la fois du premier voile et du second voile.

**6.** Construction fibreuse selon l'une quelconque des revendications 1 à 5, les premières fibres solubles dans l'eau et les secondes fibres solubles dans l'eau étant indépendamment sélectionnées parmi la méthylcellulose, l'hydroxypropyl méthylcellulose, la gomme de guar, l'alginate, la polyvinyl pyrrolidone, le poly(oxyde d'éthylène), le poly(alcool de vinyle), le pullulane, le poly(acide aspartique), le poly(acide lactique), le poly(acide acrylique), leurs copolymères ou leurs mélanges.

**7.** Construction fibreuse selon l'une quelconque des revendications 1 à 6, dans laquelle les premières fibres solubles dans l'eau et les secondes fibres solubles dans l'eau présentent une cristallinité de 20 à 54 %.

**8.** Construction fibreuse selon l'une quelconque des revendications 1 à 7, dans laquelle l'agent oxydant est sélectionné parmi les peroxydes, le permanganate, le chromate, le dichromate, le tétroxyde d'osmium, le perchlorate, le nitrate de potassium, les sels perborates, les sels percarbonates, l'oxyde nitreux, l'oxyde d'argent ou leurs mélanges.

**9.** Construction fibreuse selon les revendications 1, 2 ou 3, ladite construction comprenant:

a) un substrat ester;
b) un premier voile comprenant une pluralité de premières fibres solubles dans l'eau et une perhydrolase, où la perhydrolase est encapsulée dans les premières fibres solubles dans l'eau et est présente en une quantité de 0,1 à 40 % en pds sur la base du poids total du premier voile;
c) un second voile comprenant une pluralité de secondes fibres solubles dans l'eau et du peroxyde d'hydrogène, où le peroxyde d'hydrogène est complexé sur au moins une partie du second voile;

où les secondes fibres solubles dans l'eau sont la polyvinyl pyrrolidone ou ses copolymères; et
où les premières fibres solubles dans l'eau et les secondes fibres solubles dans l'eau sont des fibres solubles dans l'eau filées en solution.

**10.** Construction fibreuse selon la revendication 9, dans laquelle les premières fibres solubles dans l'eau et les troisièmes fibres solubles dans l'eau sont indépendamment sélectionnées parmi la méthylcellulose, l'hydroxypropyl méthylcellulose, la gomme de guar, l'alginate, la polyvinyl pyrrolidone, le poly(oxyde d'éthylène), le poly(alcool de vinyle), le pullulane, le poly(acide aspartique), le poly(acide lactique), le poly(acide acrylique), leurs copolymères ou leurs mélanges.

**11.** Construction fibreuse selon la revendication 9, dans laquelle le substrat ester est absorbé sur au moins une partie du premier voile.

**12.** Construction fibreuse selon la revendication 9, dans laquelle les premières fibres solubles dans l'eau sont sélectionnées parmi la méthylcellulose, l'hydroxypropyl méthylcellulose, la gomme de guar, l'alginate, la polyvinyl pyrrolidone, le poly(oxyde d'éthylène), le poly(alcool de vinyle), le pullulane, le poly(acide aspartique), le poly(acide lactique), le poly(acide acrylique), leurs copolymères ou leurs mélanges.

**13.** Construction fibreuse selon l'une quelconque des revendications précédentes, dans laquelle le substrat ester est sélectionné parmi la diacétine, la triacétine, l'acétate d'éthyle, le lactate d'éthyle ou leurs mélanges.

**14.** Construction fibreuse selon l'une quelconque des revendications précédentes dans laquelle l'efficacité d'encapsulation de la perhydrolase est de 80 à 100 %.

**15.** Construction fibreuse selon l'une quelconque des revendications précédentes dans laquelle; (i) la construction fibreuse se dissout dans une solution ayant 70 % en pds d'eau ou plus basé sur le pourcentage total en poids de la solution; et/ou (ii) la perhydrolase est fournie dans une composition qui est sensiblement exempte de cellules ou de débris cellulaires; et/ou (iii) la construction fibreuse se présente sous la forme d'un voile tissé, d'un voile tissé fragmenté, d'un voile non-tissé, d'un voile non-tissé fragmenté, de fibres individuelles ou de leurs combinaisons.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010062745 A
- WO 2009067279 A
- US 7618579 B
- US 7582247 B


### Non-patent literature cited in the description


- **BRISCOE B ; LUCKHAM P ; ZHU S.** The effects of hydrogen bonding upon the viscosity of aqueous poly(vinyl alcohol) solutions. *Polymer,* 2000, vol. 41, 3851-3860
- **L. PROCHÁZKOVÁ ; Y. RODRÍGUEZ-MUÑOZ ; J. PROCHÁZKA ; J. WANNERA.** *Intern. J. Environ. Anal. Chem.,* 2014, vol. 94, 399-410
- **I.F. ALEKSANDROVICH ; L.N. LYUBIMOVA.** *Fibre Chem.,* 1993, vol. 24, 156
- **D.P. JOSHI ; Y.L. LAN-CHUN-FUNG ; J.G. PRITCHARD.** *Anal. Chim. Acta.,* 1979, vol. 104, 153
- **Y. MORISHIMA ; K. FUJISAWA ; S. NOZAKURA.** *Polym. J.,* 1978, vol. 10, 281
- **J.G. PRITCHARD ; D.A. AKINTOLA.** *Talanta,* 1972, vol. 19, 877
- **L.F. FRYER ; B.S. GUPTA.** Determination of Pore Size Distribution in Fibrous Webs and its Impact on Absorbency. *Proceedings of 1996 Nonwovens Confernce,* 1996, 321-327
- **GUPTA, B.S. ; SMITH, D. K.** Nonwovens in Absorbent Materials. *Textile Sci. and Technol.,* 2002, vol. 13, 349-388
- **G. K. SURYA PRAKASH ; ANTON SHAKHMIN ; KEVIN G. GLINTON ; SNEHA RAO ; THOMAS MATHEW ; GEORGE A. OLAH.** *Green Chem.,* 2014, vol. 16, 3616-3622
- **E. F. PANARIN ; K. K. KALNIN'SH ; V. V. AZANOVA.** *Polym. Sci. Ser. A.,* 2007, vol. 49 (3), 275-283
- **M. A. ZOLFIGOL ; G. CHEHARDOLI ; M. SHIRI.** *Reac. Func. Polym.,* 2007, vol. 67, 723-727
- **F. HAAF ; A. SANNER ; F. STRAUB.** *Polym. J.,* 1985, vol. 17 (1), 143-152
- **LAEMMLI, U. K.** Cleavage of structural proteins during the assembly of the head of bacteriophage T4. *Nature,* 1970, vol. 227 (5259), 680-685
- **PINKERNELL, U. ; EFFKEMANN, S. ; KARST, U.** Simultaneous HPLC determination of Peroxyacetic acid and hydrogen peroxide. *Anal. Chem.,* 1997, vol. 69 (17), 3623-3627
- **PINKERNELL, U. ; EFFKEMANN, S. ; KARST, U.** Simultaneous HPLC determination of peroxyacetic acid and hydrogen peroxide. *Anal. Chem.,* 1997, vol. 69, 3623-3627